# EUROPÄISCHE PATENTSCHRIFT

(11) **EP 0 758 326 B1**
(45) Veröffentlichungstag und Bekanntmachung des Hinweises auf die Patenterteilung: **31.10.2001**
(21) Anmeldenummer: 95917938.3
(22) Anmeldetag: 19.04.1995
(51) Int. Cl.: C07D 285/08, C07D 417/12, A01N 43/836

(54) **ALKYLSULFINYL- UND ALKYLSULFONYL-1,2,4-THIADIAZOLYLOXYACETAMIDE SOWIE IHRE VERWENDUNG ALS HERBIZIDE**
ALKYL SULPHINYL AND ALKYL SULPHONYL-1,2,4-THIADIAZOLYLOXY ACETAMIDES AND THEIR USE AS HERBICIDES
ALKYLSULFINYL- ET ALKYLSULFONYL-1,2,4,- THIADIAZOLYLOXYACETAMIDES ET LEUR UTILISATION COMME HERBICIDES

(30) Priorität: 02.05.1994 DE 4415338
(43) Veröffentlichungstag der Anmeldung: 19.02.1997
(73) Patentinhaber: BAYER AG, 51368 Leverkusen (DE)
(72) Erfinder: FÖRSTER, Heinz, D-56337 Kadenbach (DE); SANTEL, Hans-Joachim, D-51371 Leverkusen (DE); DOLLINGER, Markus, D-51381 Leverkusen (DE)
(86) Internationale Anmeldenummer: EP9501472
(87) Internationale Veröffentlichungsnummer: WO9529905

(56) Entgegenhaltungen:
- EP-A- 0 018 497
- EP-A- 0 029 171
- EP-A- 0 348 737
- EP-A- 0 626 380
- CHEMICAL ABSTRACTS, vol. 70, no. 17, 28. April 1969, Columbus, Ohio, US; abstract no. 77873q, T. NOGUCHI ET AL. 'Selective toxicity. VIII. Biological activities of 1,2,4-thiadiazole derivatives' Seite 344 ; & YAKUGAKU ZASSHI, Bd.88, Nr.11, 1968, JP Seiten 1437 - 1449

## Beschreibung

Die Erfindung betrifft neue Alkylsulfinyl- und Alkylsulfonyl-1,2,4-thiadiazolyloxyacetamide, Verfahren und neue Zwischenprodukte zu ihrer Herstellung und ihre Verwendung als Herbizide.

Es ist bereits bekannt, daß bestimmte Alkylsulfinyl- und Alkylsulfonyl-1,2,4-thiadiazolyloxyacetamide, wie z.B. N-Isopropyl-α-(3-methylsulfinyl-1,2,4-thiadiazol-5-yloxy)-acetanilid, herbizide Eigenschaften aufweisen (vgl. z.B. EP-A 348 737 und die vorgängige, jedoch nicht vorveröffentlichte Patentanmeldung DE-P 4 317 323 vom 25.5.1993). Die Wirksamkeit dieser vorbekannten Verbindungen ist jedoch, insbesondere bei niedrigen Aufwandmengen und Konzentrationen nicht in allen Anwendungsgebieten völlig zufriedenstellend.

Es wurden nun die neuen Alkylsulfinyl- und Alkylsulfonyl-1,2,4-thiadiazolyloxyacetamide der allgemeinen Formel (I) gefunden, 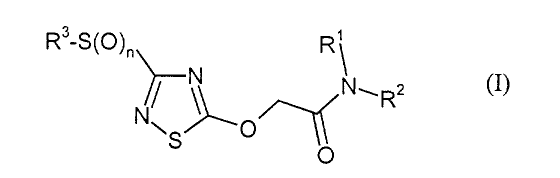 in welcher
- n: für die Zahlen 1 oder 2 steht,
- R¹: für Wasserstoff, C₁-C₈-Alkyl (welches gegebenenfalls durch Fluor, Chlor, Cyano oder C₁-C₄-Alkoxy substituiert ist), für C₂-C₈-Alkenyl (welches gegebenenfalls durch Fluor und/oder Chlor substituiert ist), für C₂-C₈-Alkinyl oder für Benzyl steht,
- R²: für C₁-C₈-Alkyl (welches gegebenenfalls durch Fluor, Chlor, Cyano oder C₁-C₄-Alkoxy substituiert ist), C₂-C₈-Alkenyl (welches gegebenenfalls durch Fluor und/oder Chlor substituiert ist), für C₂-C₈-Alkinyl, für C₃-C₆-Cycloalkyl (welches gegebenenfalls durch Chlor und/oder C₁-C₃-Alkyl substituiert ist), für C₅- oder C₆-Cycloalkenyl, für C₁-C₈-Alkoxy (welches gegebenenfalls durch C₁-C₄-Alkoxy substituiert ist), oder für C₃-C₄-Alkenyloxy steht, oder
- R¹ und R²: zusammen mit dem Stickstoffatom, an das sie gebunden sind, einen gegebenenfalls ein- bis dreifach durch C₁-C₃-Alkyl substituierten, gesättigten oder ungesättigten fünf- bis siebengliedrigen Stickstoffheterocyclus bilden, welcher gegebenenfalls benzannelliert ist, und
- R³: für C₁-C₈-Alkyl (welches gegebenenfalls durch Fluor, Chlor, Brom, Cyano, C₁-C₄-Alkoxy, Dioxolanyl oder Dioxanyl substituiert ist) oder für Phenyl oder Phenyl-C₁-C₂-alkyl (welche jeweils gegebenenfalls durch Fluor, Chlor, Brom, Cyano, Nitro, C₁-C₄-Alkyl oder C₁-C₄-Alkoxy substituiert sind) steht.

Weiter wurde gefunden daß man die neuen Alkylsulfinyl- und Alkylsulfonyl-1,2,4-thiadiazolyloxyacetamide der allgemeinen Formel (I) erhält, wenn man
(a) 1,2,4-Thiadiazol-derivate der allgemeinen Formel (II) 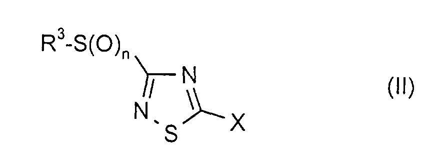 in welcher
   - X: für Halogen oder die Gruppierung -S(O)ₙ-R³ steht und
   - n und R³: die oben angegebene Bedeutung haben,
   mit Hydroxyacetamiden der allgemeinen Formel (III) 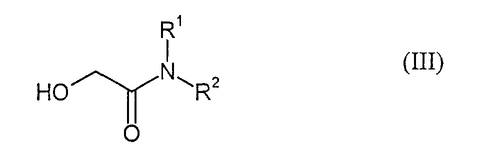 in welcher
   - R¹ und R²: die oben angegebene Bedeutung haben,
   gegebenenfalls in Gegenwart eines Verdünnungsmittels, gegebenenfalls in Gegenwart eines Säurebindemittels und gegebenenfalls in Gegenwart eines Katalysators umsetzt, oder wenn man
(b) Alkyl(Aryl-, Aralkyl-)thio-1,2,4-thiadiazolyloxyacetamide der allgemeinen Formel (IV) 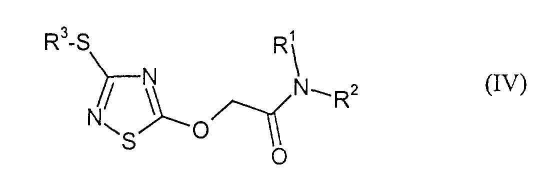 in welcher
   - R¹, R² und R³: die oben angegebene Bedeutung haben,
   mit einem Oxidationsmittel gegebenenfalls in Gegenwart eines Katalysators und gegebenenfalls in Gegenwart eines Verdünnungsmittels umsetzt.

Schließlich wurde gefunden, daß die neuen Alkylsulfinyl- und Alkylsulfonyl-1,2,4-thiadiazolyloxyacetamide der allgemeinen Formel (I) interessante herbizide Eigenschaften besitzen.

Überraschenderweise zeigen die erfindungsgemäßen Verbindungen der Formel (I) bei teilweise sehr guter Verträglichkeit gegenüber Kulturpflanzen, wie z.B. Baumwolle, erheblich stärkere Wirkung gegen schwer bekämpfbare Unkräuter als die chemisch ähnliche bekannte Verbindung N-Isopropyl-α-(3-methylsulfinyl-1,2,4-thiadiazol-5-yl-oxy)-acetanilid.

Die Erfindung betrifft insbesondere Verbindungen der Formel (I), in welcher
- n: für die Zahlen 1 oder 2 steht,
- R¹: für Methyl, Ethyl, n- oder i-Propyl, n-, i- oder s-Butyl, n-, i- oder s-Pentyl (welche jeweils gegebenenfalls durch Fluor, Chlor, Cyano, Methoxy oder Ethoxy substituiert sind), für Propenyl, Butenyl, Propinyl oder Butinyl steht,
- R²: für Methyl, Ethyl, n- oder i- Propyl, n-, i- oder s-Butyl, n-, i- oder s-Pentyl, n-, i- oder s-Hexyl (welche jeweils gegebenenfalls durch Fluor, Chlor, Cyano, Methoxy oder Ethoxy substituiert sind), für Propenyl, Butenyl, Pentenyl, Propinyl, Butinyl oder Pentinyl, für Cyclopentyl oder Cyclohexyl (welche jeweils gegebenenfalls durch Methyl und/oder Ethyl substituiert sind), für Cyclohexenyl, für Methoxy, Ethoxy, n- oder i-Propoxy, n-, i- oder s-Butoxy, n-, i- oder s-Pentyloxy (welche jeweils gegebenenfalls durch Methoxy oder Ethoxy subtituiert sind) steht, oder
- R¹ und R²: zusammen mit dem Stickstoffatom, an das sie gebunden sind, für gegebenenfalls ein- bis dreifach durch Methyl und/oder Ethyl substituiertes Piperidinyl, für gegebenenfalls ein- oder zweifach durch Methyl und/oder Ethyl substituiertes Pyrrolidinyl, für Perhydroazepinyl oder für 1,2,3,4-Tetrahydro(iso)-chinolinyl stehen, und
- R³: für Methyl, Ethyl, n- oder i-Propyl, n-, i-, s- oder t-Butyl, n-, i-, s- oder t-Pentyl (welche jeweils gegebenenfalls durch Fluor, Chlor, Cyano, Methoxy oder Ethoxy substituiert sind) oder für Phenyl oder Benzyl (welche jeweils gegebenenfalls durch Fluor, Chlor, Cyano, Methyl oder Methoxy substituiert sind) steht,
mit Ausnahme der oben durch Disclaimer ausgenommenen Verbindungen.

Beispiele für die möglichen Bedeutungen der Gruppierung 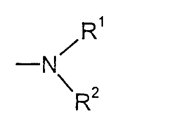 in der Formel (I) sind in der nachstehenden Tabelle 1 aufgeführt.

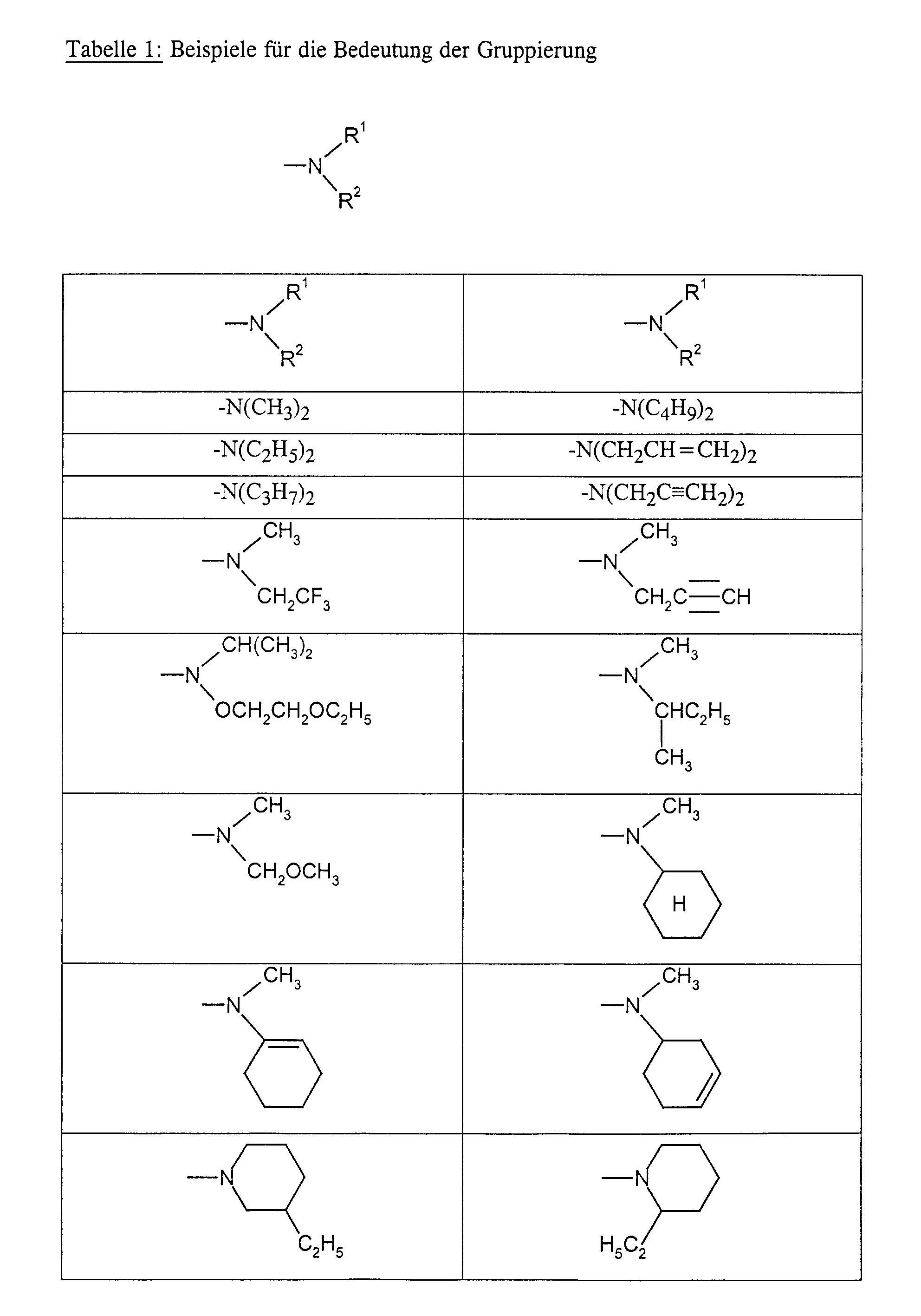

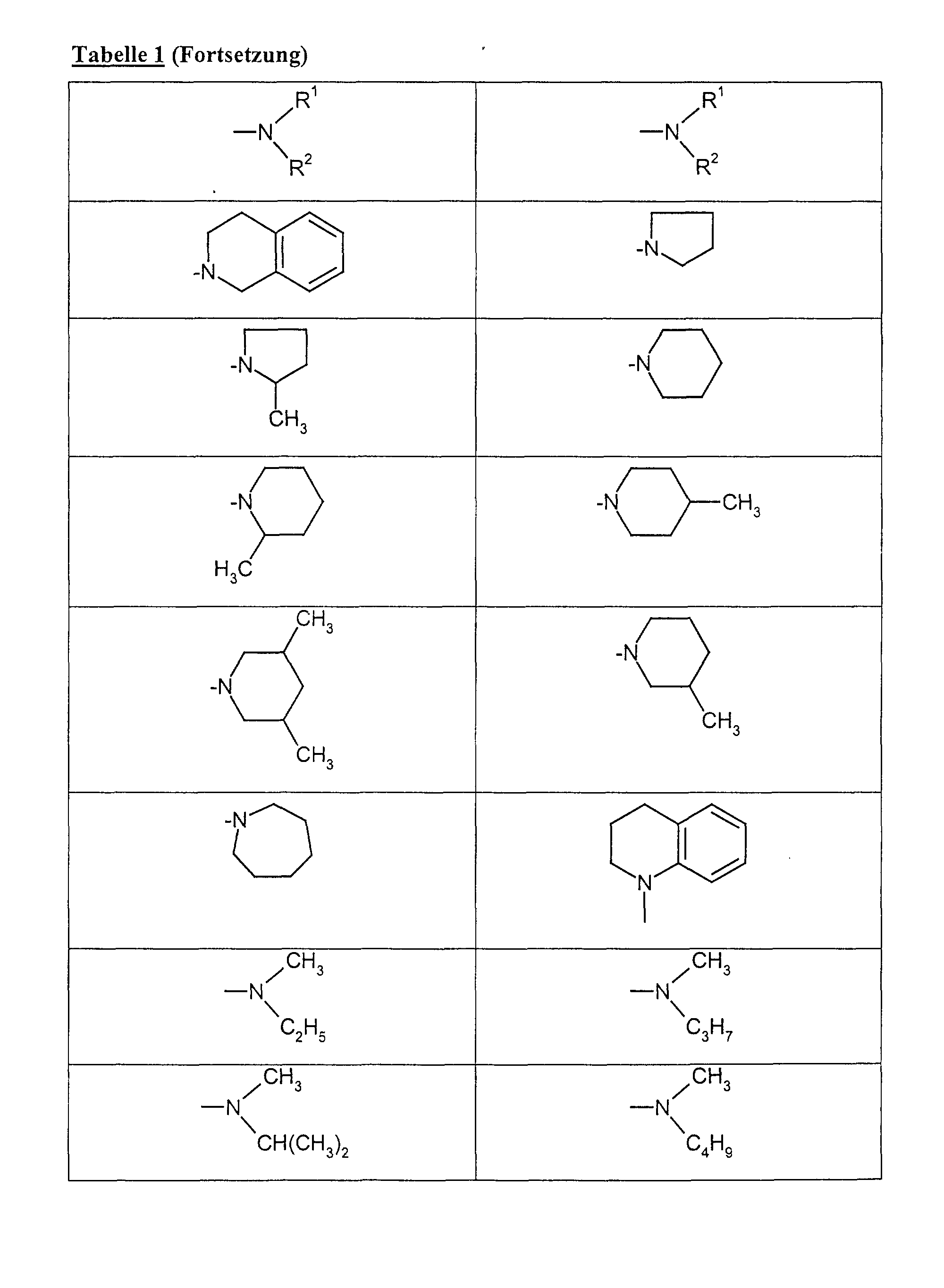

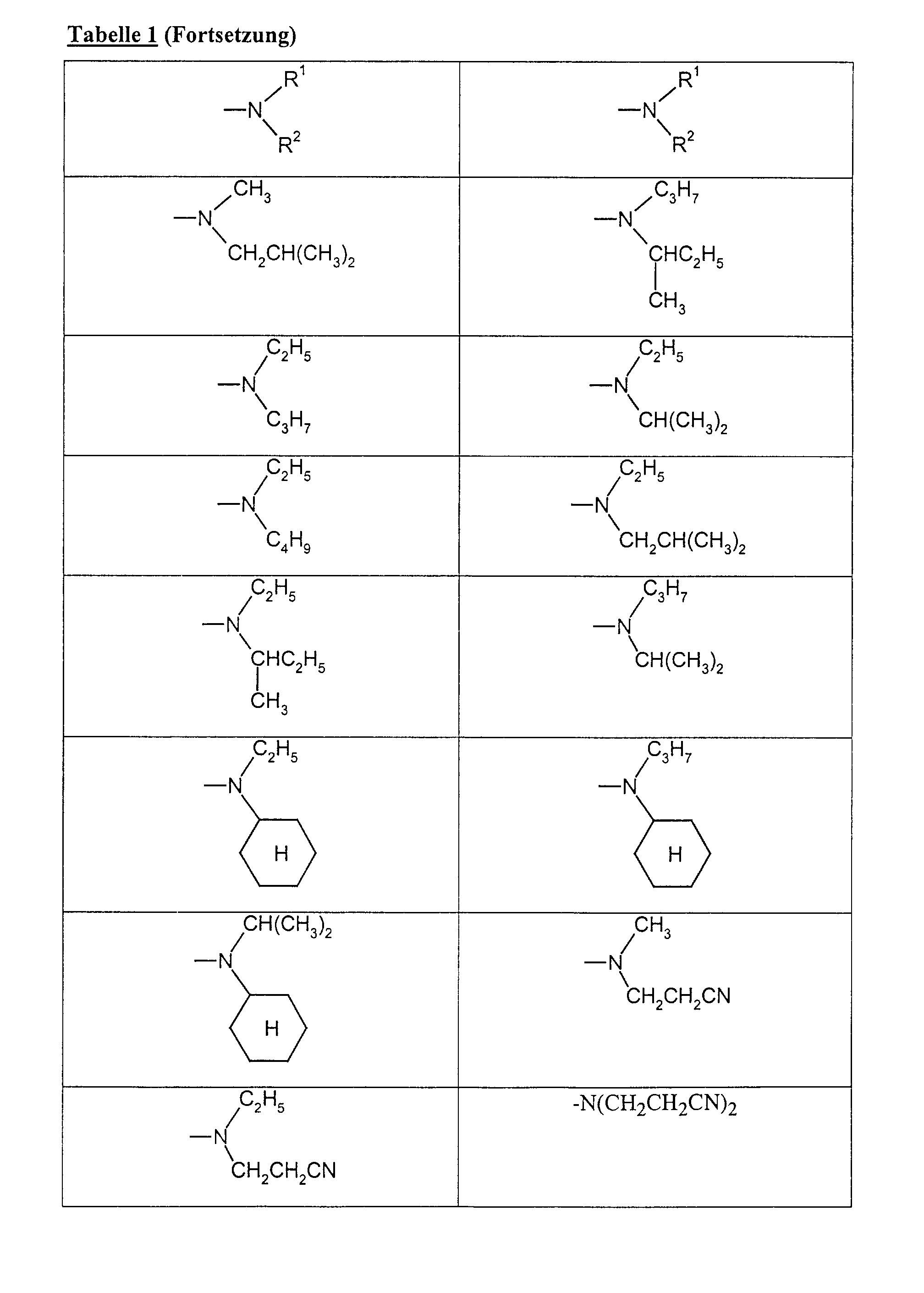

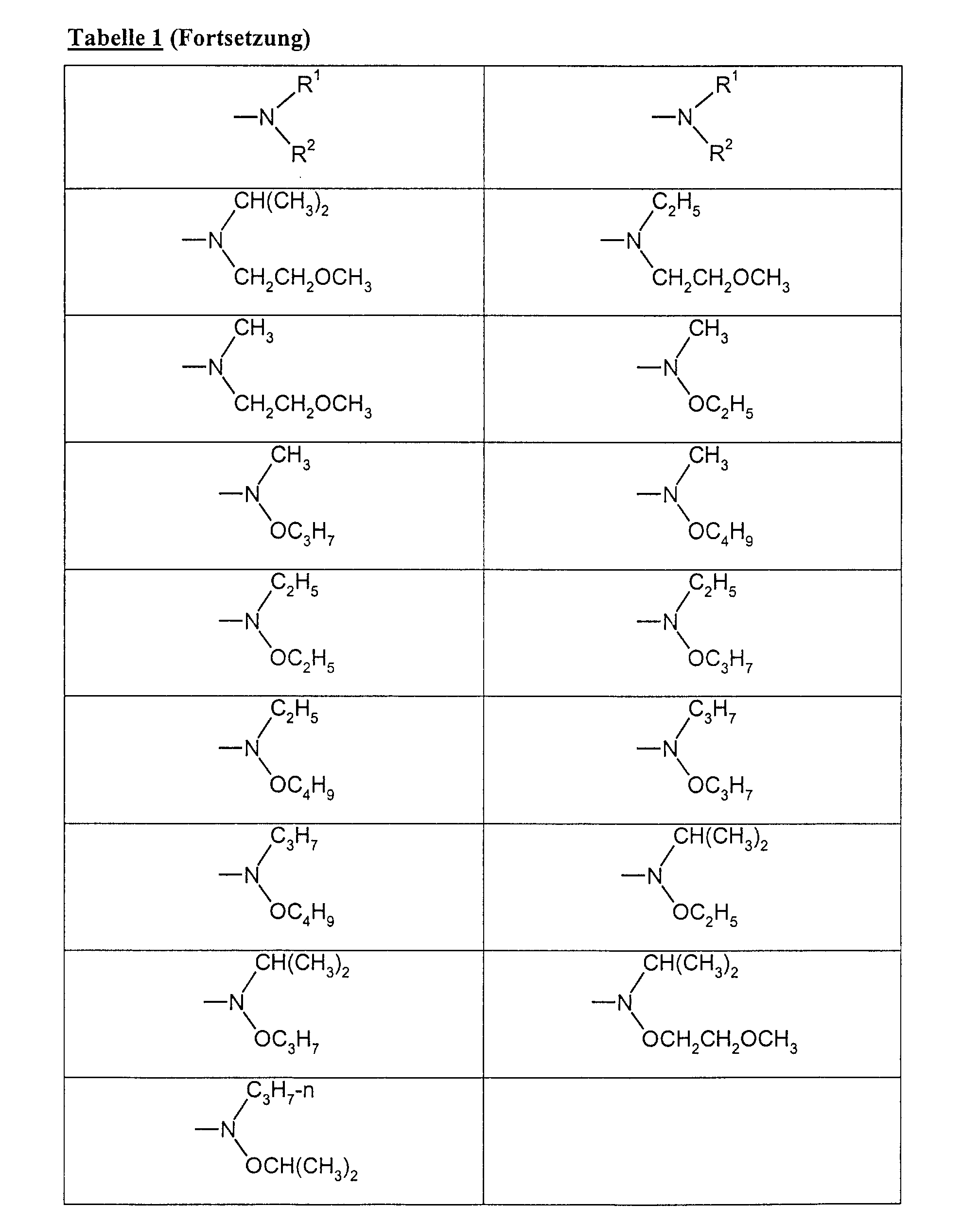

Die oben aufgeführten allgemeinen oder in Vorzugsbereichen angegebenen Restedefinitionen gelten sowohl für die Endprodukte der Formel (I) als auch entsprechend für die jeweils zu Herstellung benötigten Ausgangsstoffe bzw. Zwischenprodukte.

Diese Restedefinitionen können untereinander, also auch zwischen den angegebenen Bereichen bevorzugter Verbindungen, beliebig kombiniert werden.

Verwendet man beispielsweise N-Cyclohexyl-N-methyl-α-(3-methylthio-1,2,4-thiadiazol-5-yl-oxy)-acetamid und Hydrogenperoxid als Ausgangsstoffe, so kann der Reaktionsablauf beim erfindungsgemäßen Verfahren (b) durch das folgende Formelschema skizziert werden:

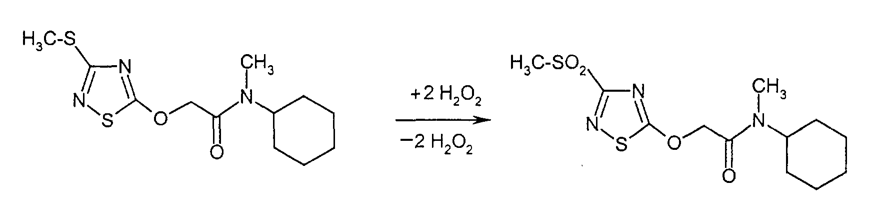

Die beim erfindungsgemäßen Verfahren (a) zur Herstellung der Verbindungen der allgemeinen Formel (I) als Ausgangsstoffe zu verwendenden 1,2,4-Thiadiazolderivate sind durch die Formel (II) allgemein definiert. In der Formel (II) haben n und R³ vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für n und R³ angegeben wurden.

Die Ausgangsstoffe der Formel (II) sind mit Ausnahme der Verbindungen 5-Chlor-3-methylsulfonyl-1,2,4-thiadiazol und 3,5-Bis-methylsulfonyl-1,2,4-thiadiazol (vgl. Chem. Ber. 97 (1964), 225-237; DE-OS 1544505) noch nicht aus der Literatur bekannt und sind mit Ausnahme der oben genannten Verbindungen ebenfalls Gegenstand der vorliegenden Anmeldung.

Man erhält die 1,2,4-Thiadiazol-derivate der Formel (II), wenn man entsprechende Alkylthioverbindungen der Formel (V) 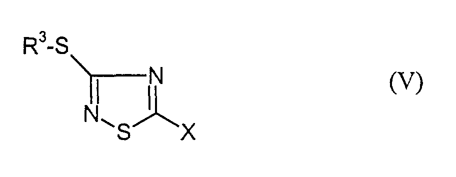 in welcher
R³ und X die oben angegebene Bedeutung haben,
mit einem Oxidationsmittel, wie z.B. Hydrogenperoxid, gegebenenfalls in Gegenwart eines Reaktionshilfsmittels, wie z.B. Natriumwolframat oder Schwefelsäure, und gegebenenfalls in Gegenwart eines Verdünnungsmittels, wie z.B. Wasser, Methanol, Ameisensäure und/oder Essigsäure, bei Temperaturen zwischen 0°C und 150°C umsetzt (vgl. die Herstellungsbeispiele).

Die Vorprodukte der Formel (V) sind bekannt und/oder können nach an sich bekannten Verfahren hergestellt werden (vgl. Chem. Ber. 90 (1957), 892-901; loc. cit. 97 (1964), 225-237; DD-PS 221060; Herstellungsbeispiele).

Die beim erfindungsgemäßen Verfahren (a) zur Herstellung von Verbindungen der Formel (I) weiter als Ausgangsstoffe zu verwendenden Hydroxyessigsäureamide sind durch die Formel (III) allgemein definiert.

In Formel (III) haben R¹ und R² insbesondere diejenigen Bedeutungen, die bereits oben im Zusammenhang mit der Beschreibung der erfindungsgemäßen Verbindungen der Formel (I) als insbesondere bevorzugt für R¹ und R² angegeben wurden.

Die Hydroxyessigsäureamide der Formel (III) sind bekannt und/oder können nach an sich bekannten Verfahren hergestellt werden (vgl. US-P 4509971 und US-P 4 645 525; ferner US-P 4 334 073, DE-OS 30 38 598, DE-OS 30 38 636, EP-A 37 526, EP-A 348 737, DE-OS 38 19 477).

Die beim erfindungsgemäßen Verfahren (b) zur Herstellung der Verbindungen der allgemeinen Formel (I) als Ausgangsstoffe zu verwendenden Alkyl (Aryl-, Aralkyl-)-thio-1,2,4-thiadiazolyloxyacetamide sind durch die Formel (IV) allgemein definiert. In der Formel (IV) haben R¹, R² und R³ vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für R¹, R² und R³ angegeben wurden.

Die Ausgangsstoffe der Formel (IV) sind bekannt und/oder können nach an sich bekannten Verfahren hergestellt werden (vgl. EP-A 018 497, EP-A 029 171).

Das erfindungsgemäße Verfahren (a) zur Herstellung der neuen Alkylsulfinyl- und Alkylsulfonyl-1,2,4-thiadiazolyloxyacetamide der Formel (I) wird vorzugsweise unter Verwendung von Verdünnungsmitteln durchgeführt. Hierzu gehören vorzugsweise Kohlenwasserstoffe, wie z.B. Toluol, Xylol oder Cyclohexan, Halogenkohlenwasserstoffe, wie z.B. Methylenchlorid, Ethylenchlorid, Chloroform oder Chlorbenzol, Ether, wie z.B. Diethylether, Dipropylether, Diisopropylether, Dibutylether, Diisobutylether, Glycoldimethylether, Tetrahydrofuran und Dioxan, Alkohole, wie z.B. Methanol, Ethanol, Propanol, Isopropanol, Butanol, Isobutanol, sec-Butanol oder tert-Butanol, Ketone, wie z.B. Aceton, Methylethylketon, Methylisopropylketon und Methylisobutylketon, Ester, wie z.B. Essigsäuremethylester und Essigsäureethylester, Amide, wie z.B. Dimethylformamid, Dimethylacetamid und N-Methylpyrrolidon, Nitrile, wie z.B. Acetonitril und Propionitril, Sulfoxide, wie z.B. Dimethylsulfoxid sowie Wasser oder wäßrige Salzlösungen.

Als Salze verwendet man hierbei vorzugsweise Chloride oder Sulfate von Alkali- oder Erdalkalimetallen, wie beispielsweise Natriumchlorid, Kaliumchlorid oder Calciumchlorid. Besonders bevorzugt ist Natriumchlorid.

Das erfindungsgemäße Verfahren (a) wird vorteilhaft unter Verwendung von Säurebindemitteln durchgeführt. Als solche werden vorzugsweise stark basische Alkali- und Erdalkalimetallverbindungen, beispielsweise Oxide, wie z.B. Natrium-, Kalium-, Magnesium- und Calciumoxid, Hydroxide, wie z.B. Natrium-, Kalium-, Magnesium- und Calciumhydroxid, Alkoholate, wie z.B. Natrium- und Kalium-tert-butylat und/- oder Carbonate, wie z.B. Natrium-, Kalium-, Magnesium- und Calciumcarbonat verwendet.

Der Zusatz von 0,01 bis 10 Gew. -% (bezogen auf eingesetztes Glycolsäureamid der Formel (III)) eines Phasentransferkatalysators mag sich in einigen Fällen als vorteilhaft erweisen. Als Beispiele für solche Katalysatoren seien genannt:

Tetrabutylammoniumchlorid, Tetrabutylammoniumbromid, Tributyl-methylphosphoniumbromid, Trimethyl-C₁₃/C₁₅-alkyl-ammoniumchlorid, Dibenzyl-dimethylammonium-methylsulfat, Dimethyl-C₁₂/C₁₄-alkyl-benzylammoniumchlorid, Tetrabutylammoniumhydroxid, 18-Krone-6, Triethylbenzylammoniumchlorid, Trimethylbenzylammoniumchlorid, Tetraethylammoniumbromid.

Die Reaktionstemperaturen können bei dem erfindungsgemäßen Verfahren (a) in einem größeren Bereich variiert werden. Im allgemeinen arbeitet man bei Temperaturen zwischen -50°C und +110°C, vorzugsweise bei Temperaturen zwischen -20°C und +80°C.

Das erfindungsgemäße Verfahren (a) wird im allgemeinen bei Normaldruck durchgeführt; es kann aber auch bei erhöhtem oder vermindertem Druck, etwa zwischen 0,1 und 10 bar, durchgeführt werden.

Zur Durchführung des erfindungsgemäßen Verfahrens (a) setzt man je Mol 1,2,4-Thiadiazol-derivat der Formel (II) im allgemeinen 0,5 bis 5 Mol, vorzugsweise 0,8 bis 1,5 Mol, Hydroxyessigsäureamid der Formel (III) ein. Die Reaktionskomponenten können in beliebiger Reihenfolge zusammengegeben werden. Man rührt jeweils das Reaktionsgemisch bis zum Ende der Umsetzung und arbeitet nach üblichen Methoden auf (vgl. die Herstellungsbeispiele).

Das erfindungsgemäße Verfahren (b) zur Herstellung der Verbindungen der Formel (I) wird unter Verwendung eines Oxidationsmittels durchgeführt. Es kommen hierbei die üblichen zur Oxidation von organischen Sulfiden (Thioethern) zu entsprechenden Sulfoxiden oder Sulfonen geeigneten Chemikalien in Betracht. Als Beispiele für geeignete Oxidationsmittel seien genannt: Hydrogenperoxid (H₂O₂), Perameisensäure, Peressigsäure, Perpropionsäure, Perbenzoesäure und 3-Chlor-perbenzoesäure sowie Chlor oder hypochlorige Säure und deren Alkalimetall- oder Erdalkalimetallsalze.

Das erfindungsgemäße Verfahren (b) wird gegebenenfalls in Gegenwart eines Katalysators durchgeführt. Hierbei sind als Katalysatoren vorzugsweise Salze von Metallen der IV., V. und VI. Nebengruppe des Periodensystems der Elemente geeignet. Als Beispiele hierfür seien Natrium(meta)vanadat, Natriummolybdat und Natriumwolframat genannt.

Das erfindungsgemäße Verfahren (b) wird vorzugsweise unter Verwendung eines Verdünnungsmittels durchgeführt. Als Verdünnungsmittel kommen dabei neben Wasser die für Oxidationsreaktionen üblichen organischen Solventien in Betracht. Hierzu gehören vorzugsweise chlorierte Kohlenwasserstoffe, wie Methylenchlorid, Ethylenchlorid, Chloroform, Tetrachlormethan, 1,1,2-Trichlorethan, Chlorbenzol und o-Dichlorbenzol, Alkohole, wie Methanol, Ethanol, Propanol, Isopropanol, Butanol, Isobutanol und sec-Butanol, Carbonsäuren, wie Ameisensäure, Essigsäure und Propionsäure.

Analog zu den Herstellungsbeispielen 1 und 2 sowie entsprechend der allgemeinen Beschreibung der erfindungsgemäßen Herstellungsverfahren können beispielsweise auch die in der nachstehenden Tabelle 2 aufgeführten Verbindungen der Formel (I) hergestellt werden.

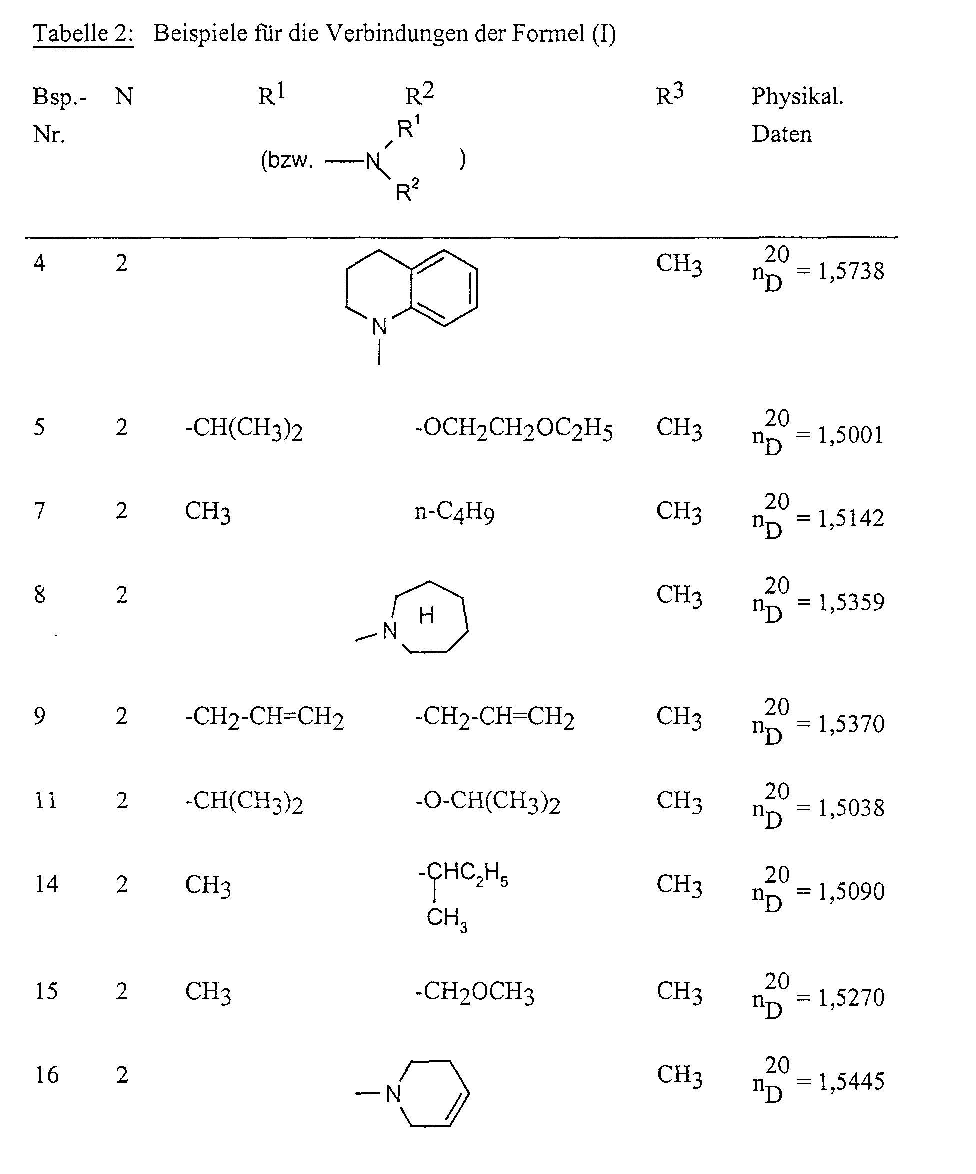

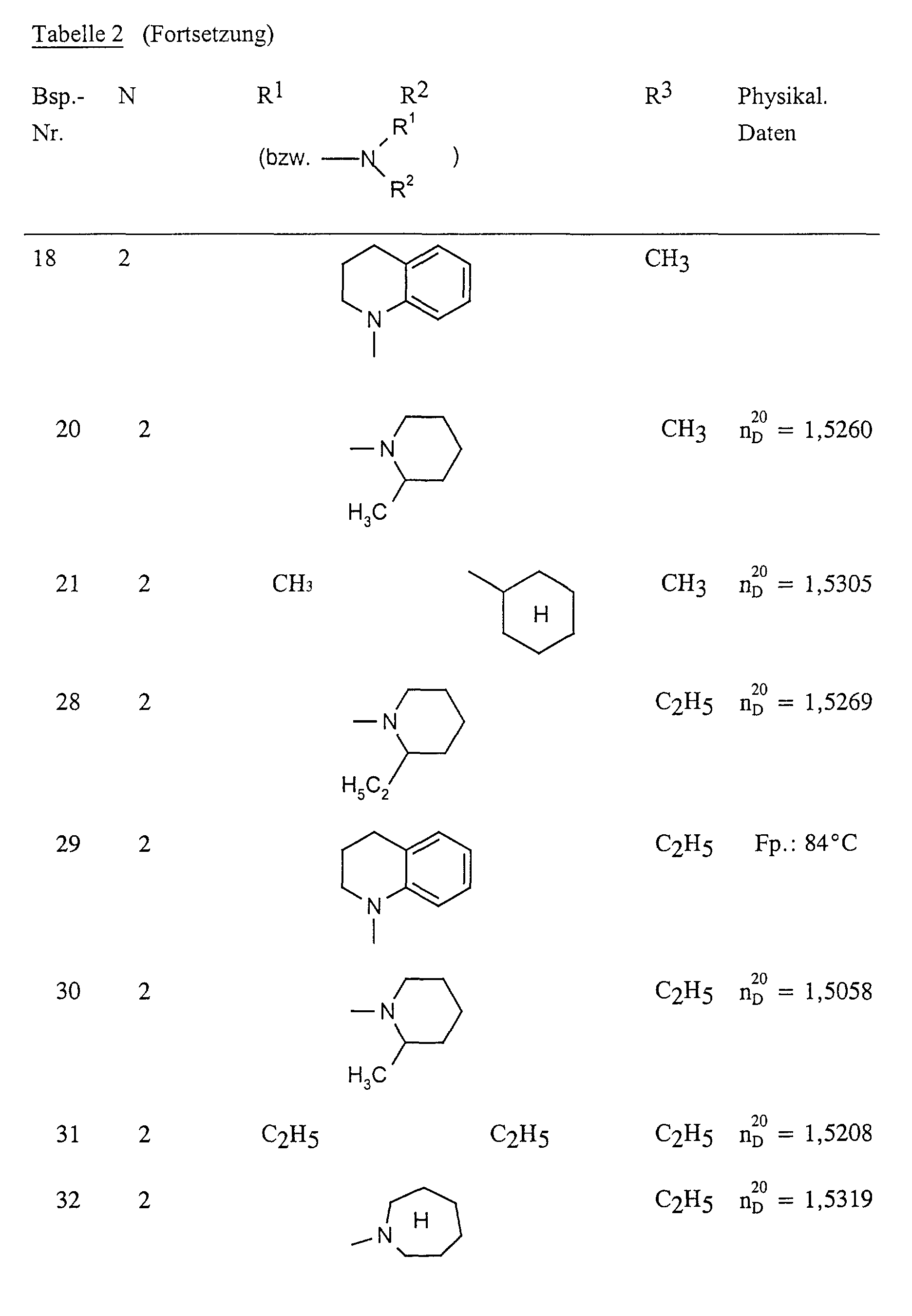

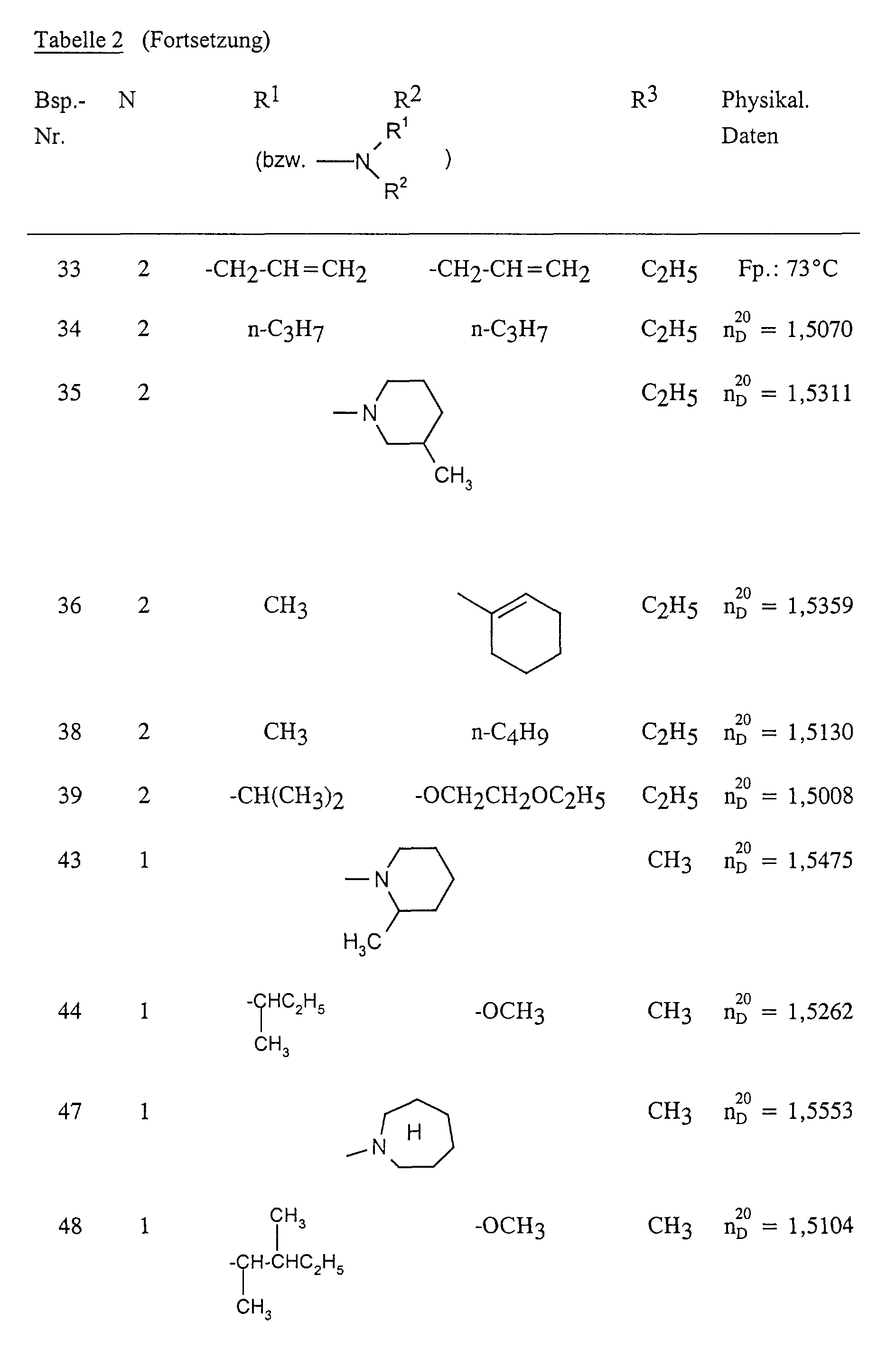

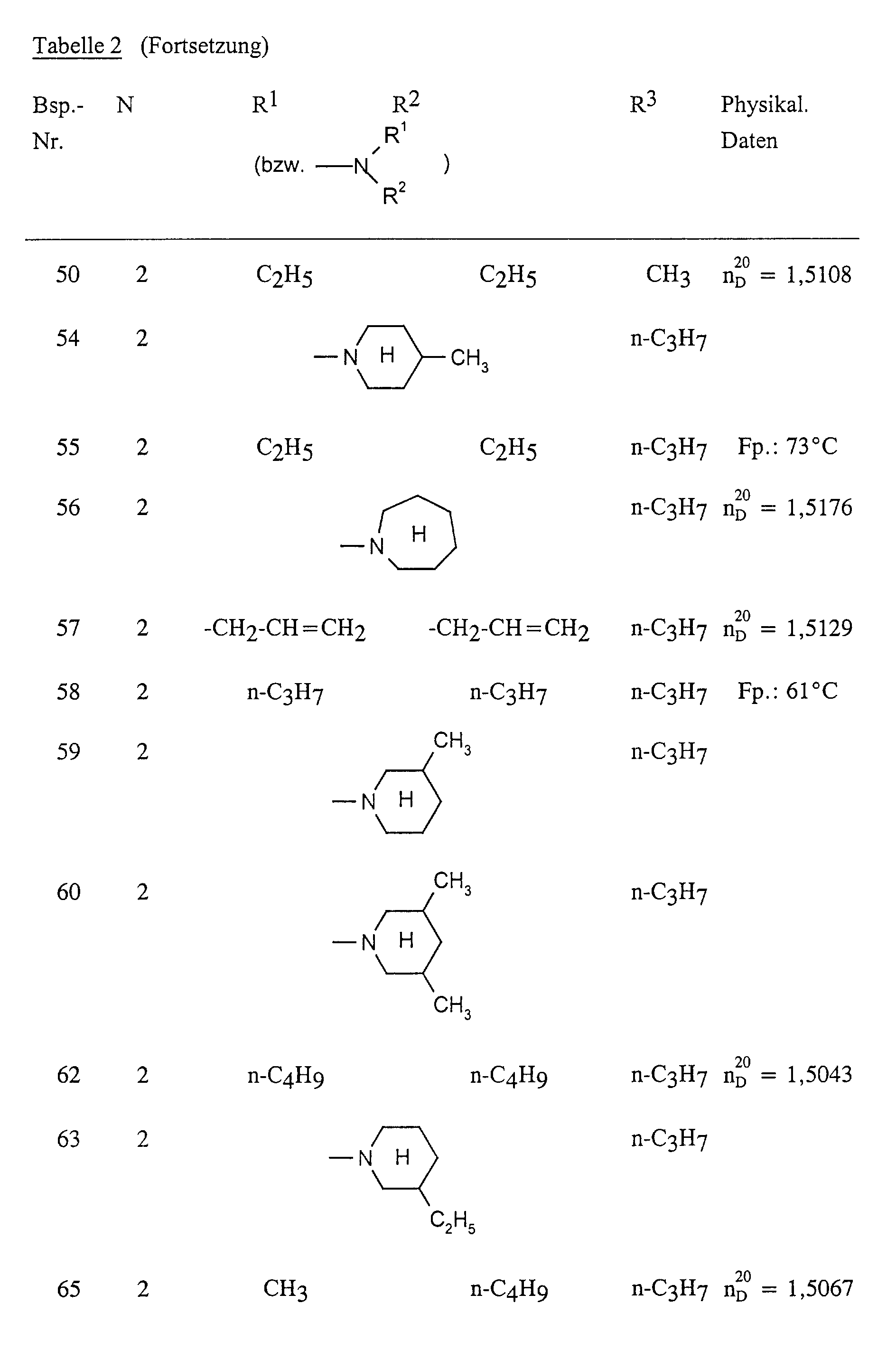

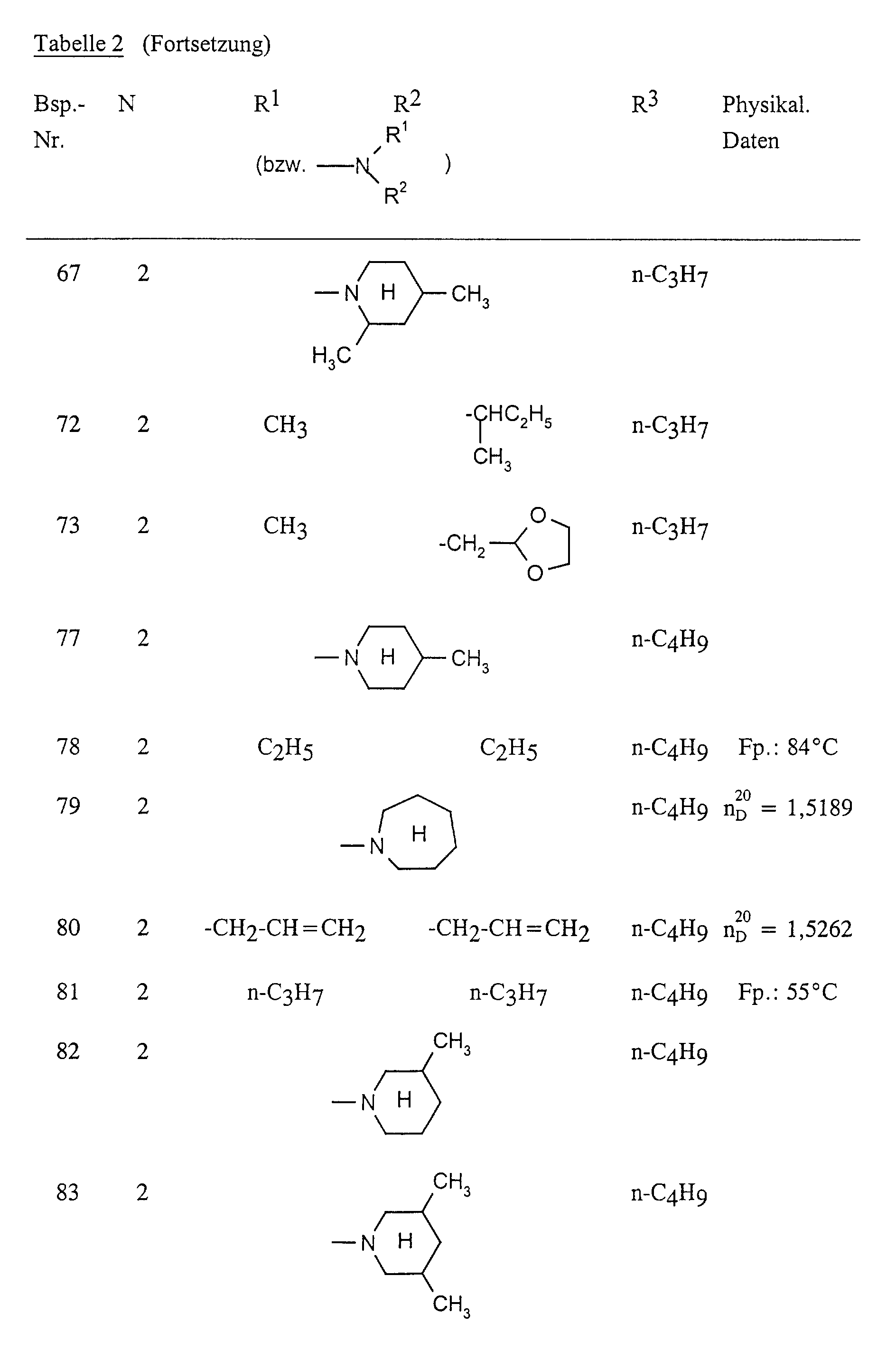

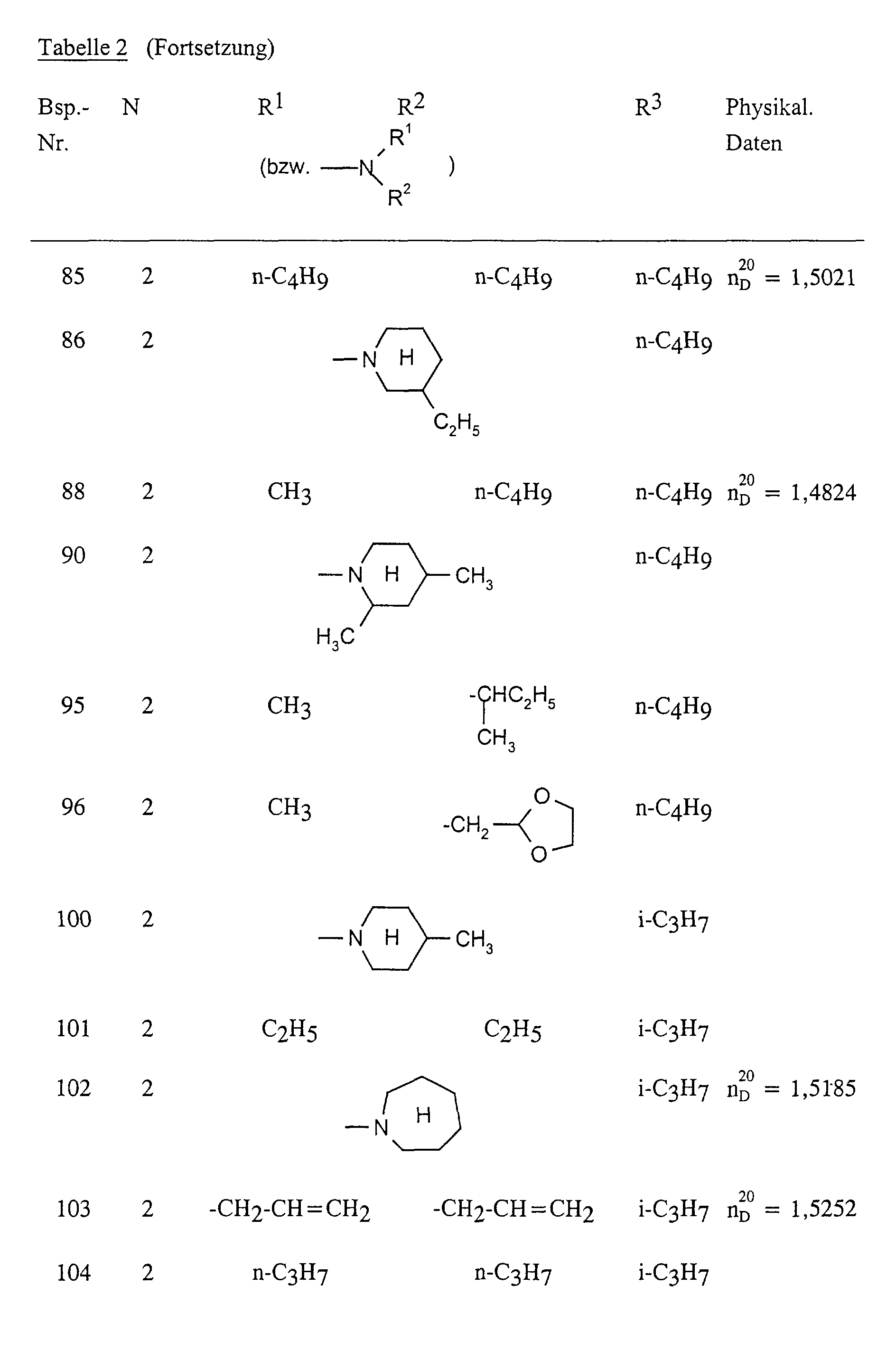

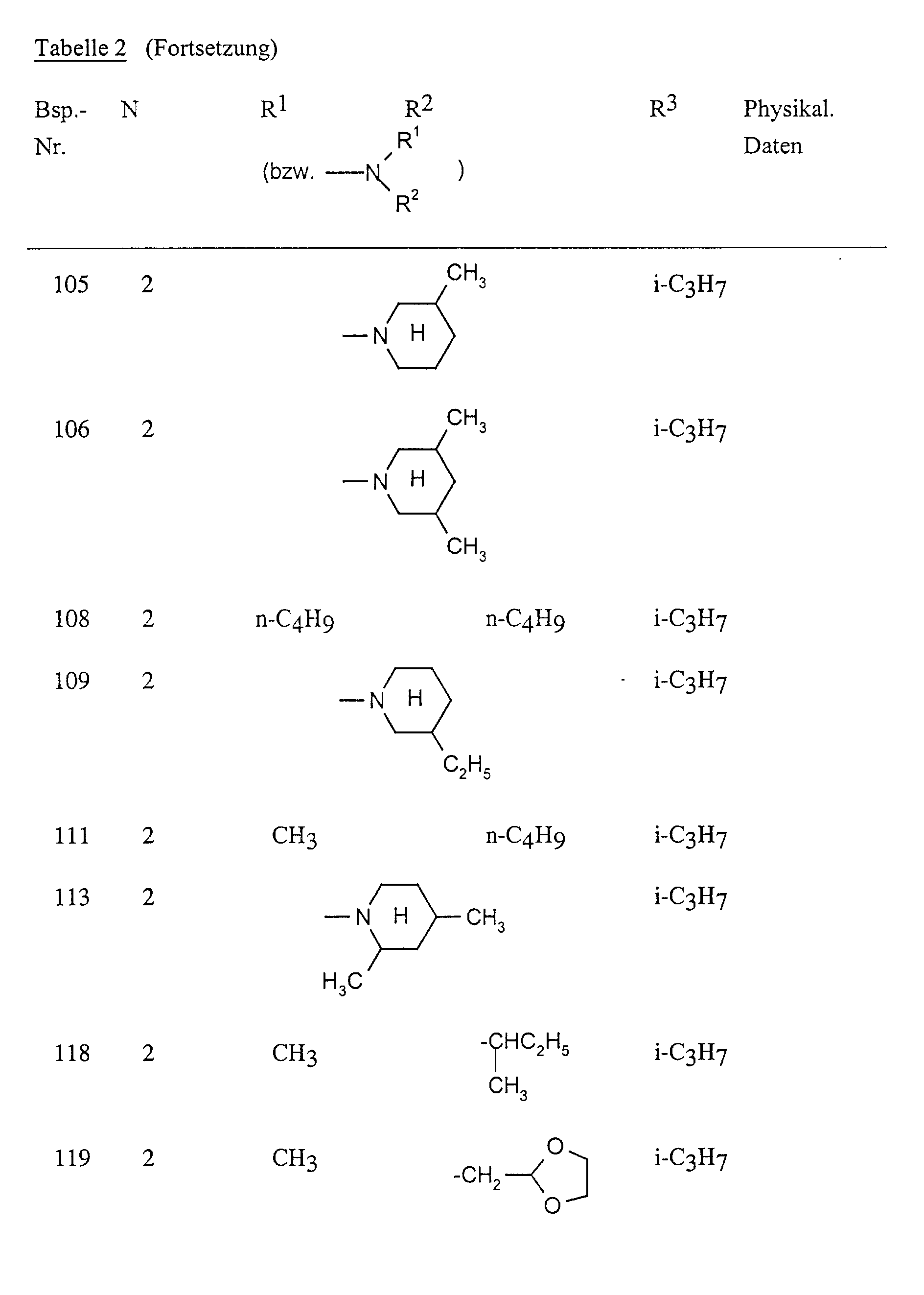

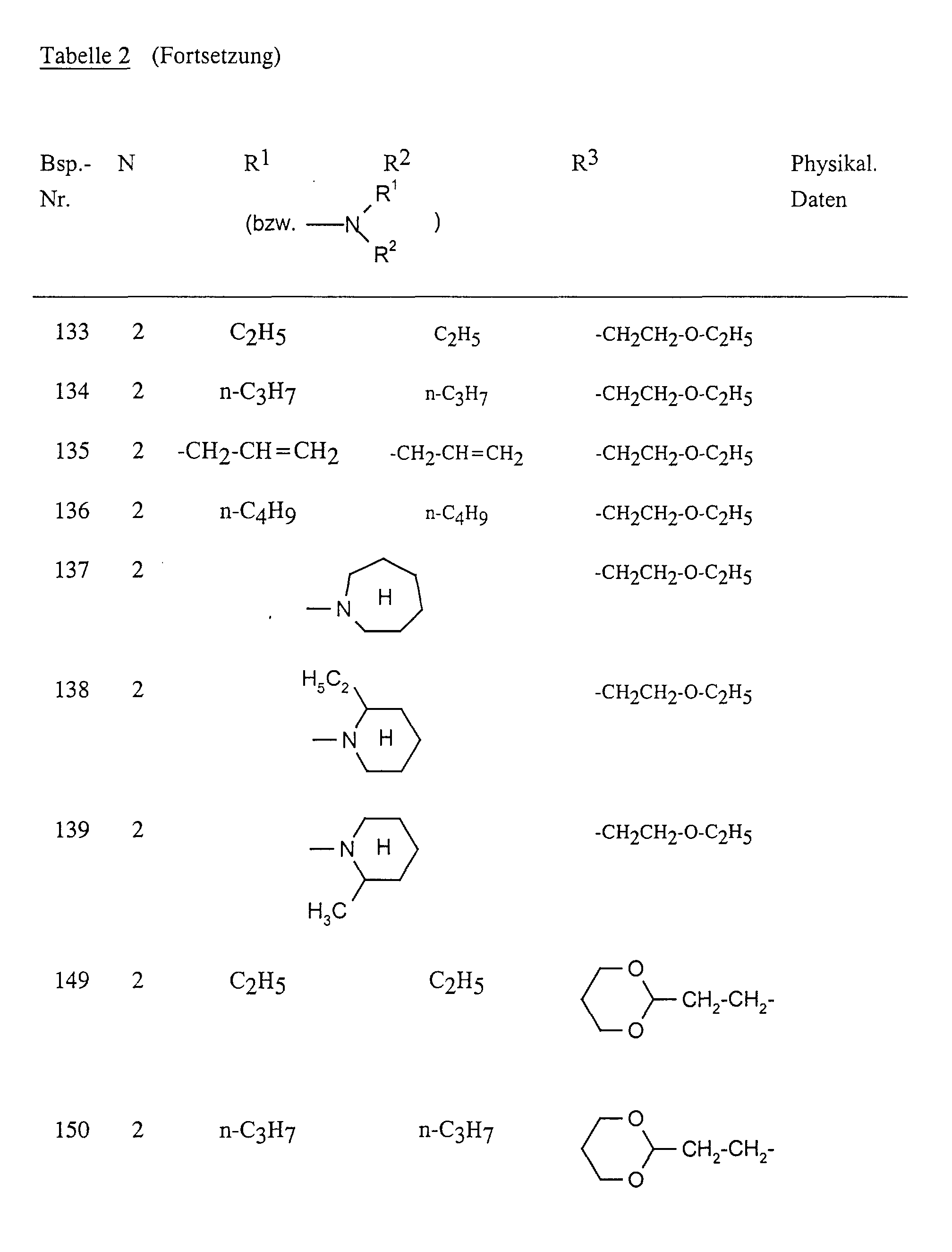

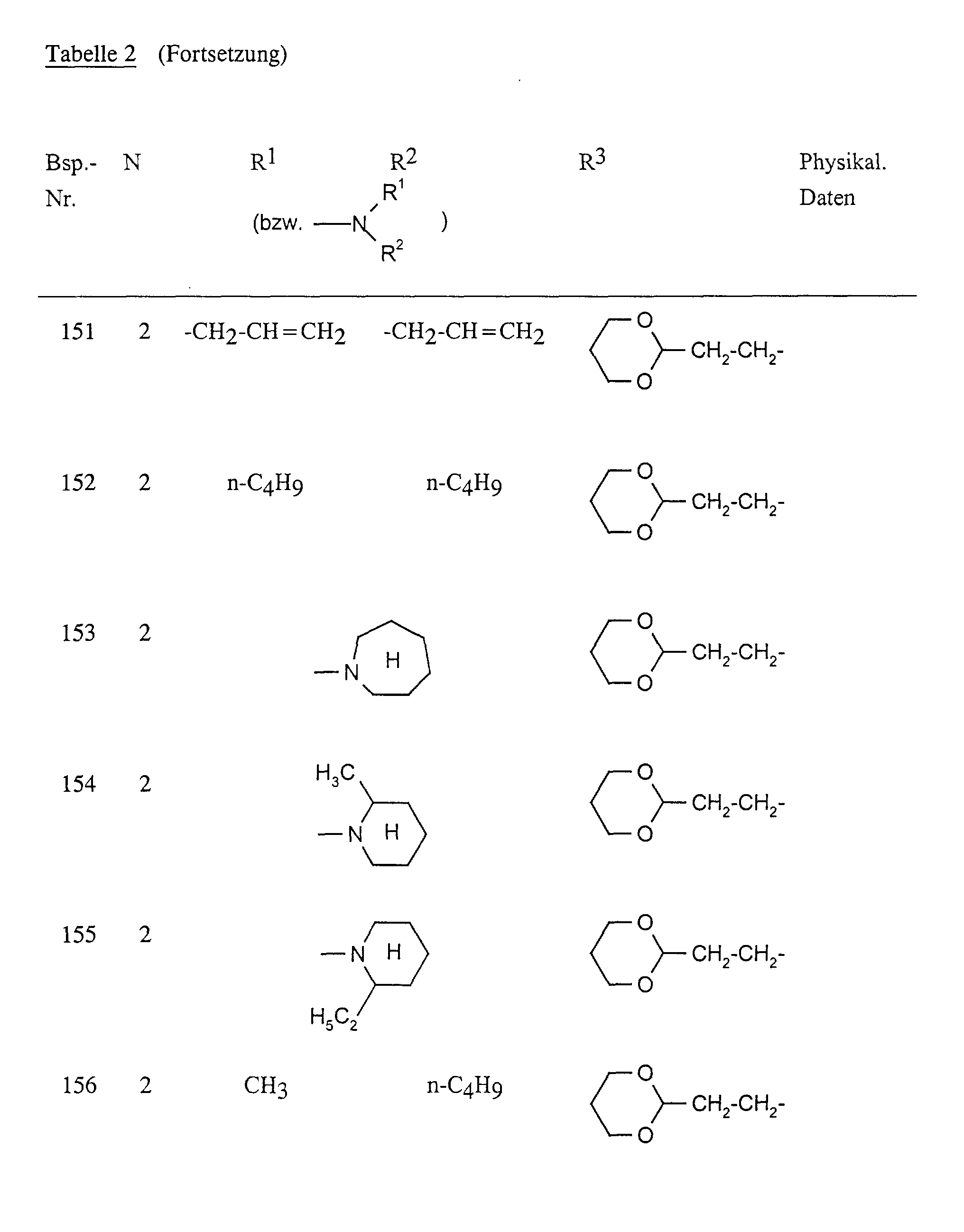

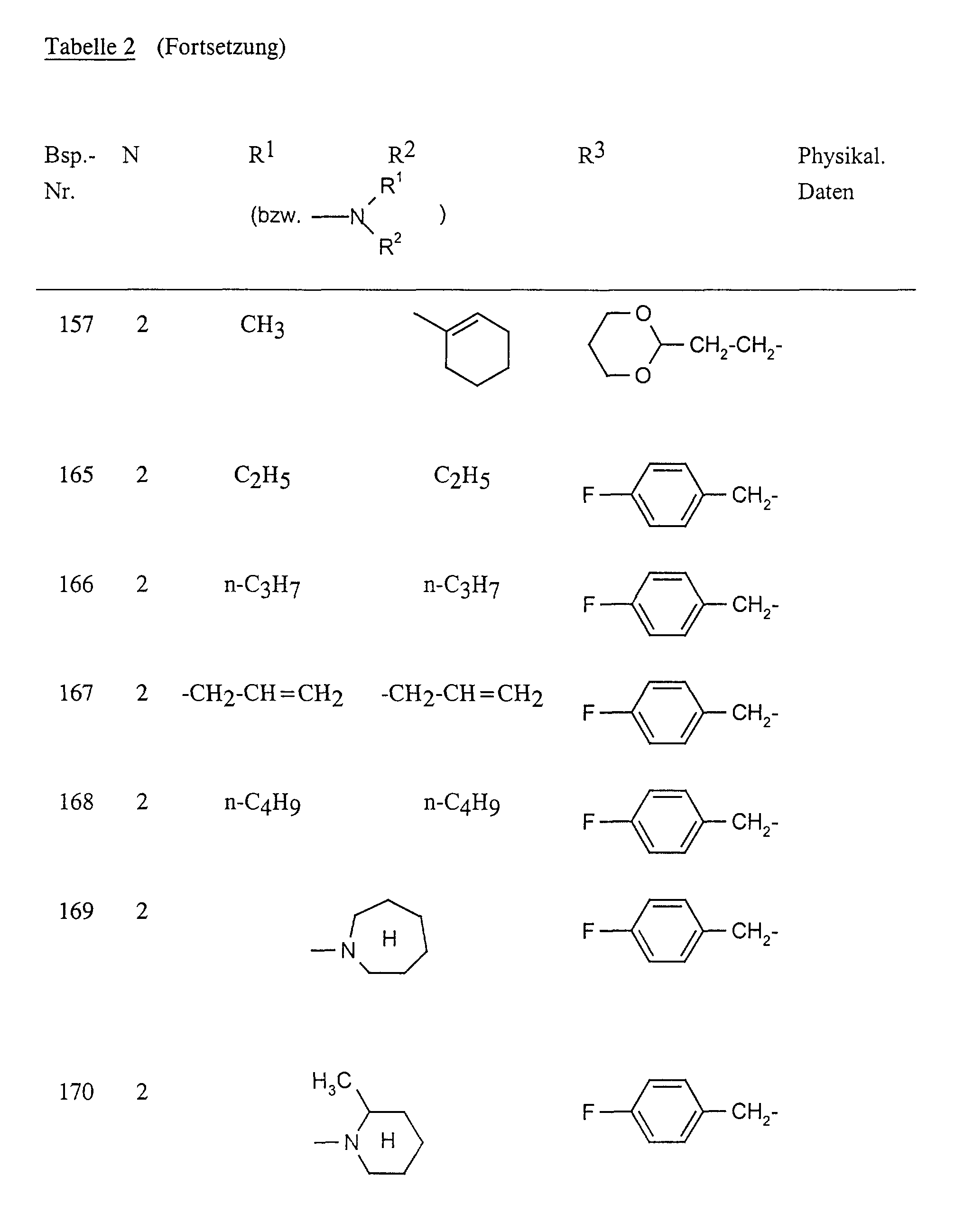

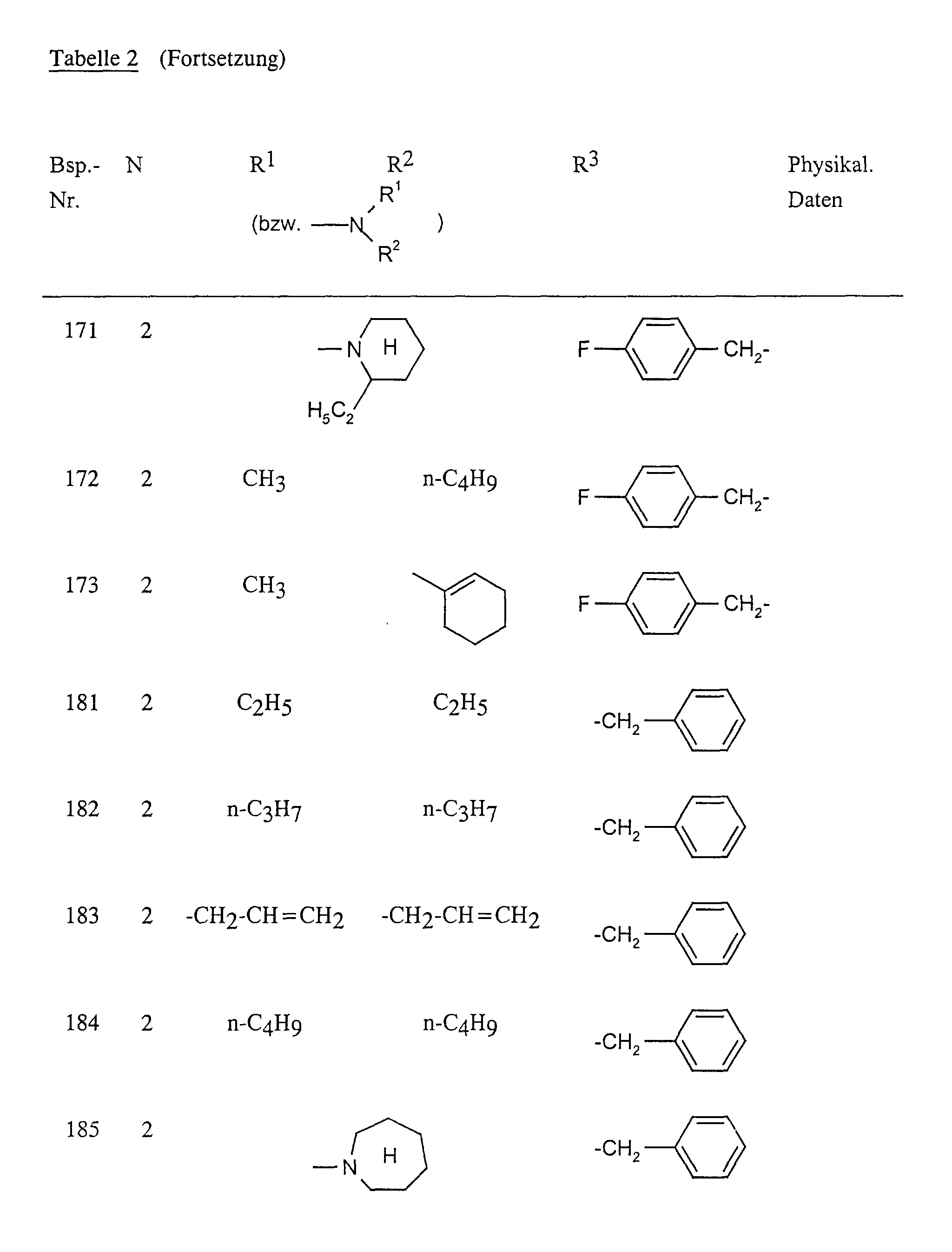

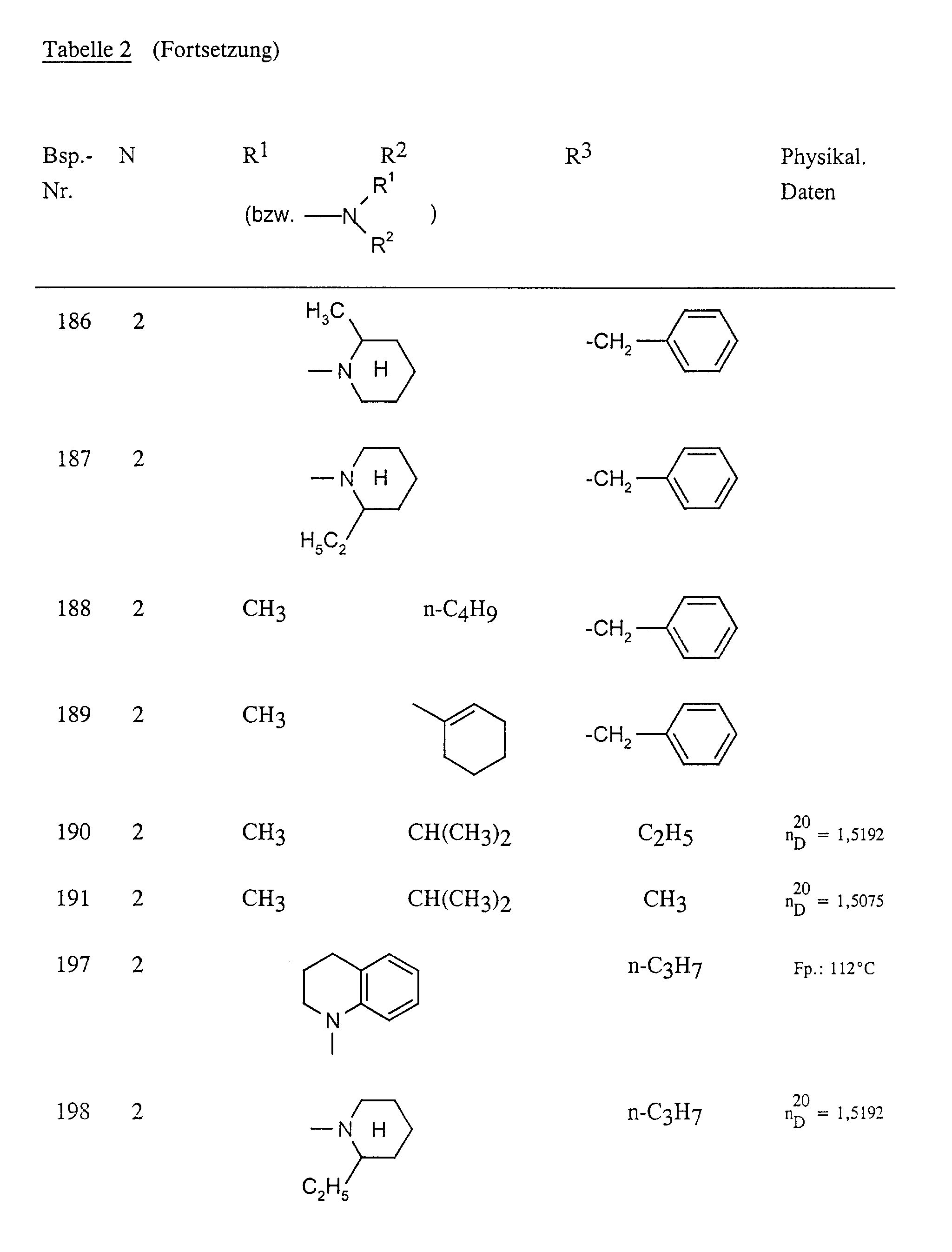

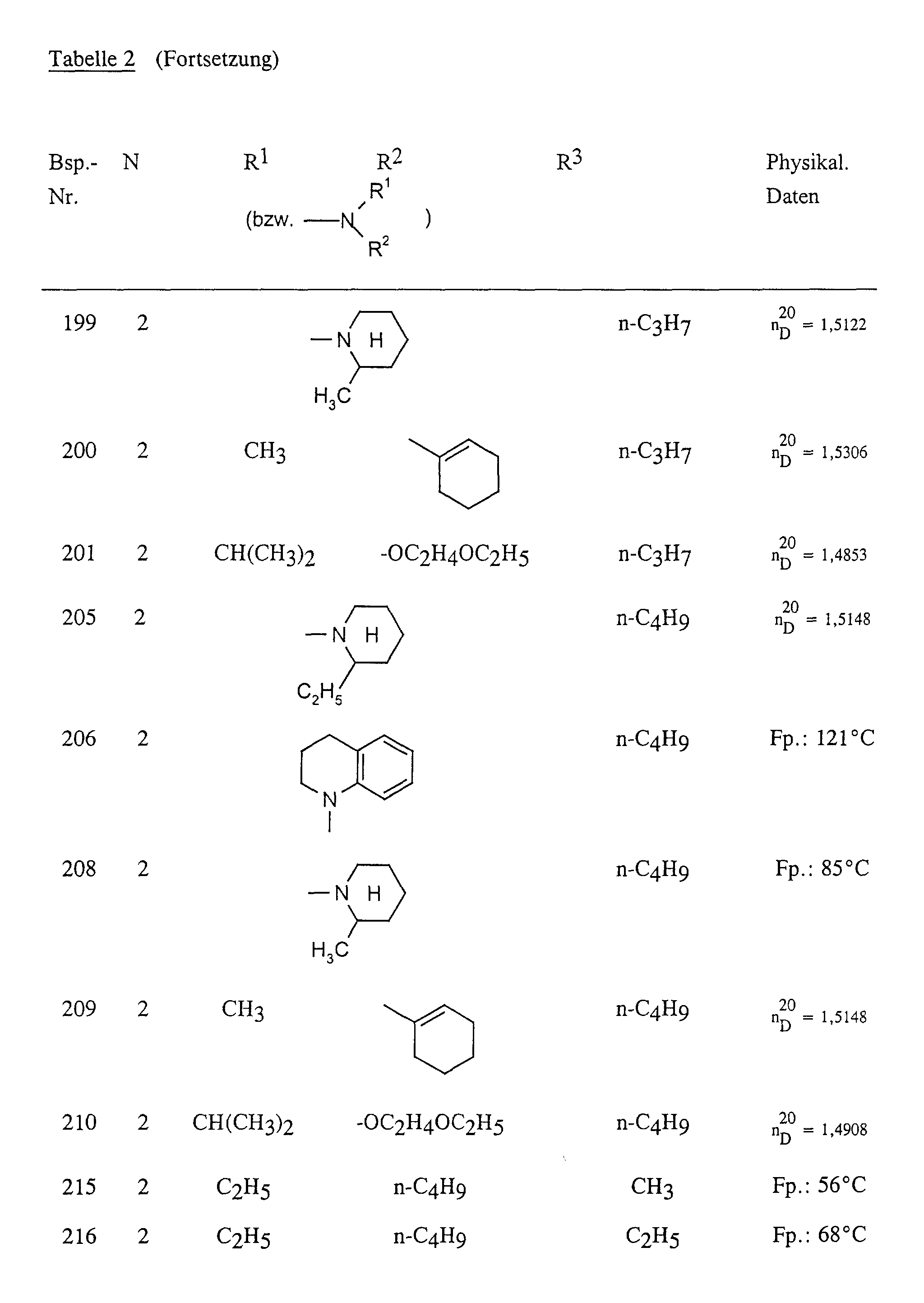

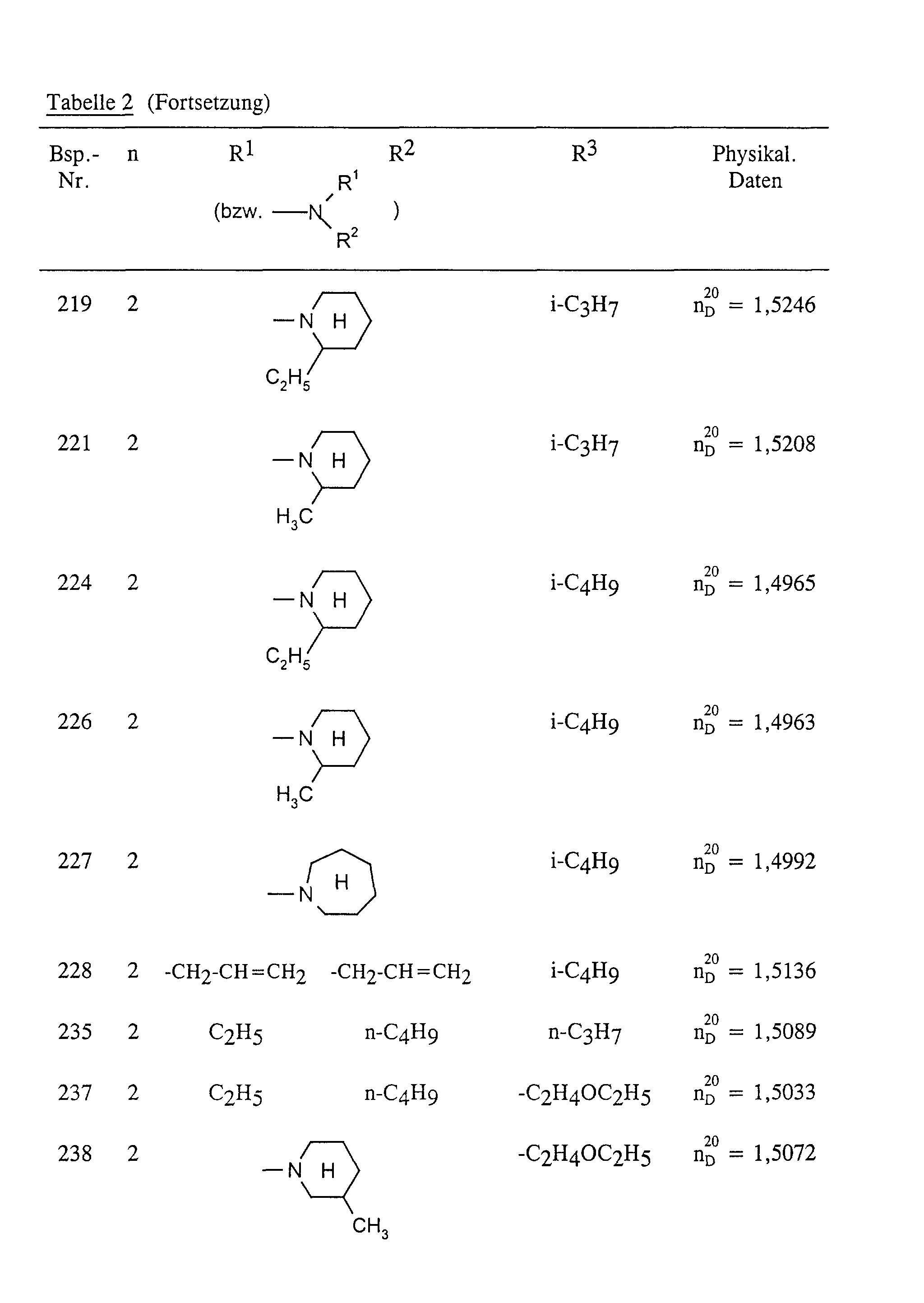

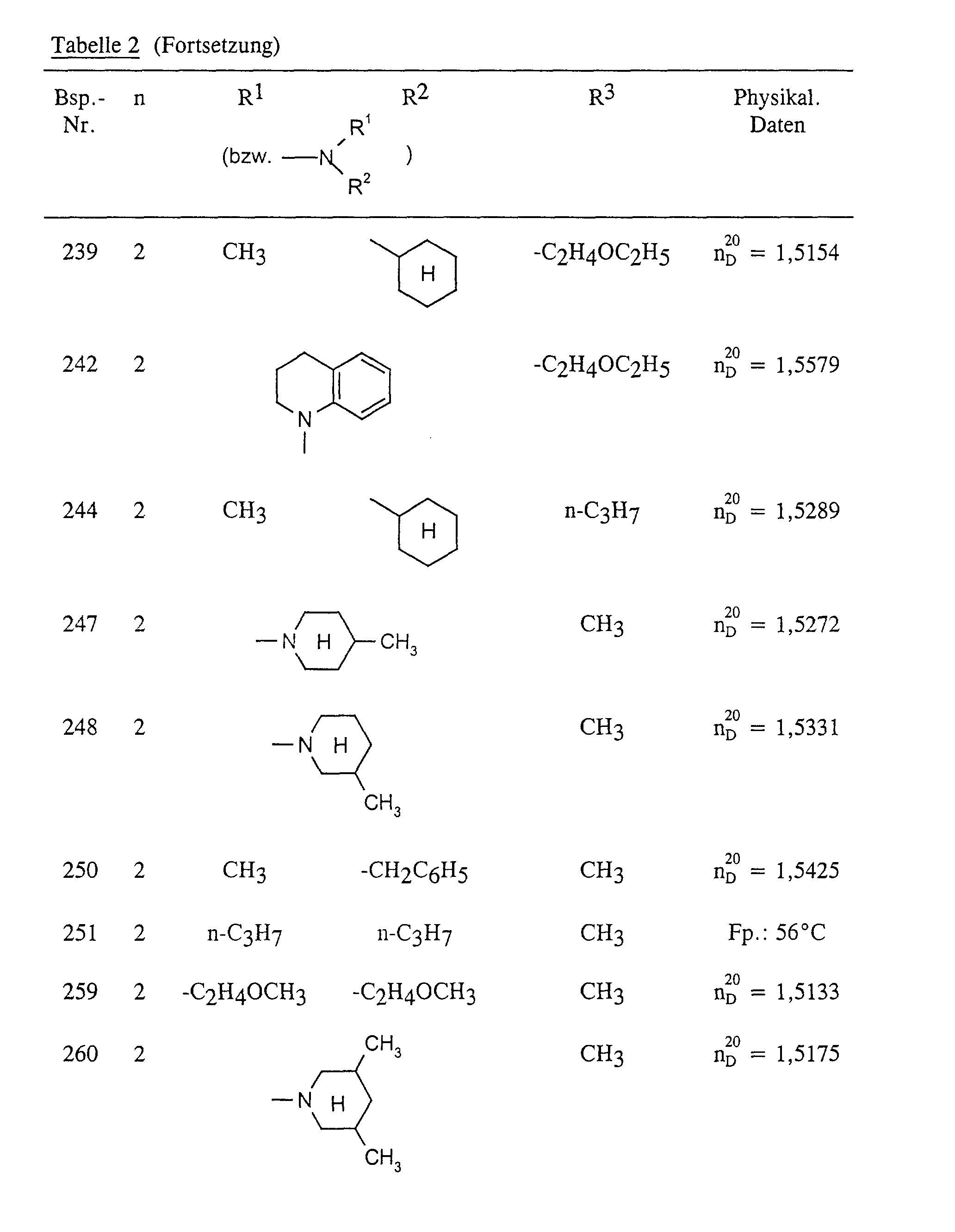

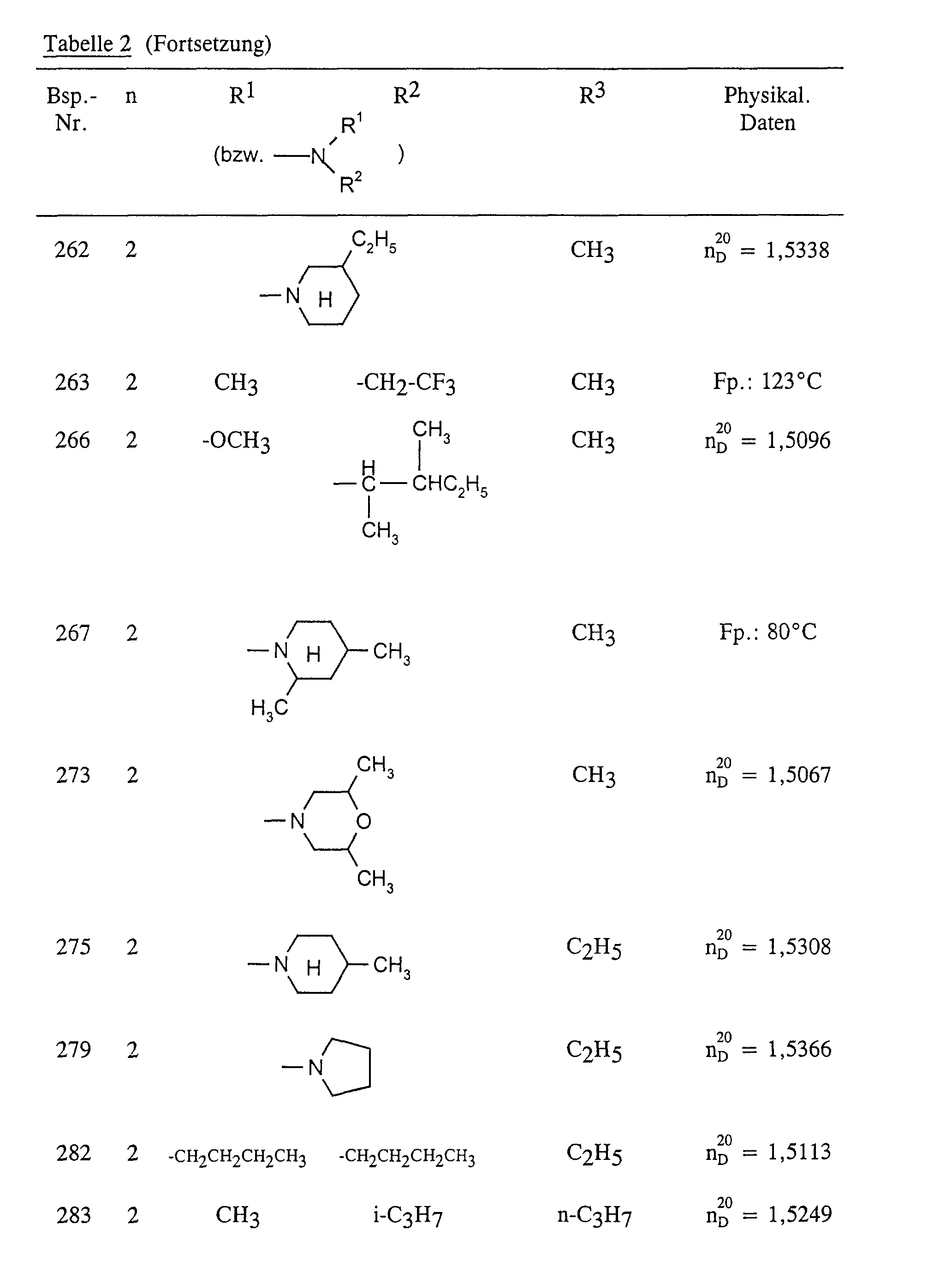

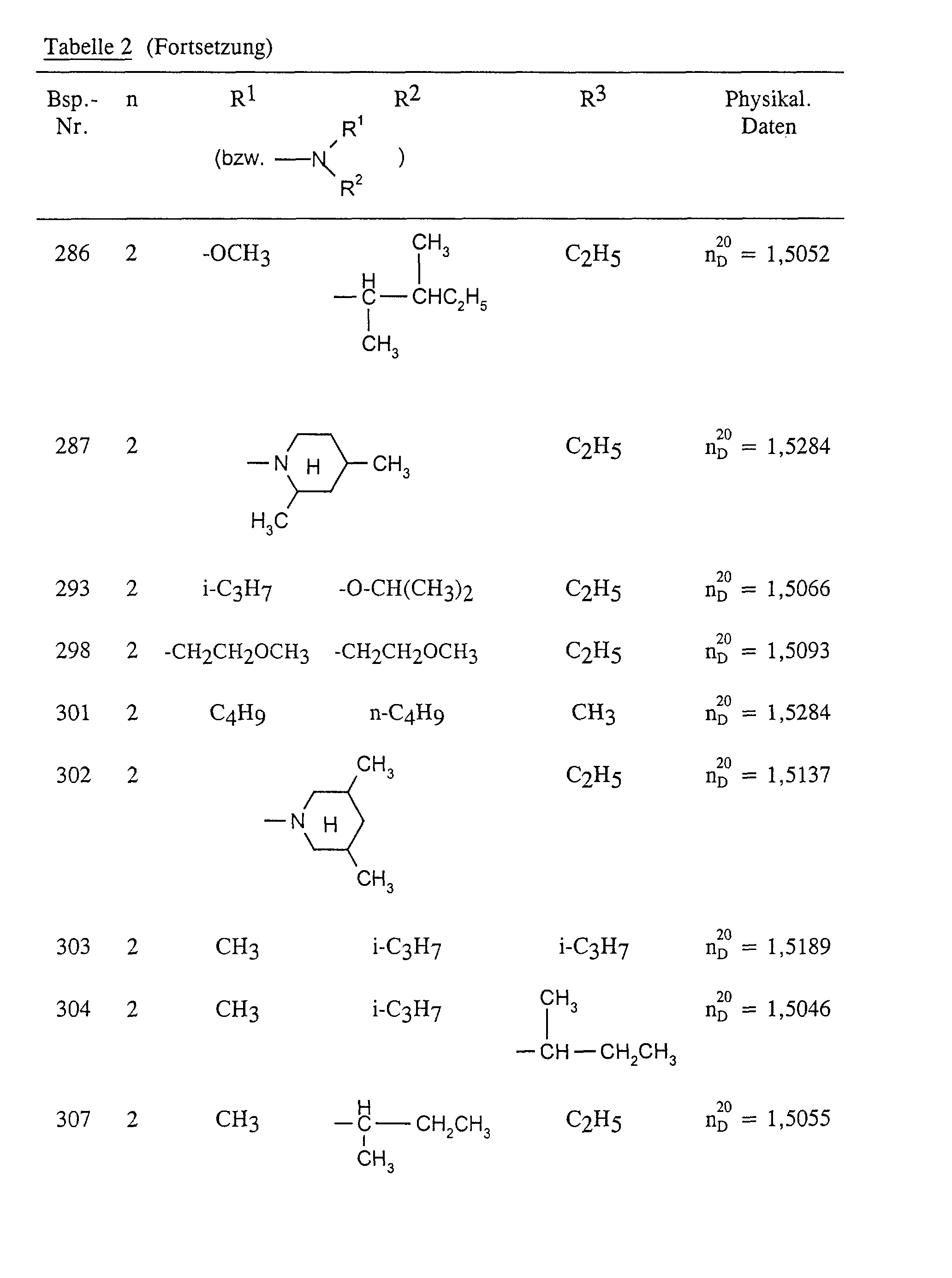

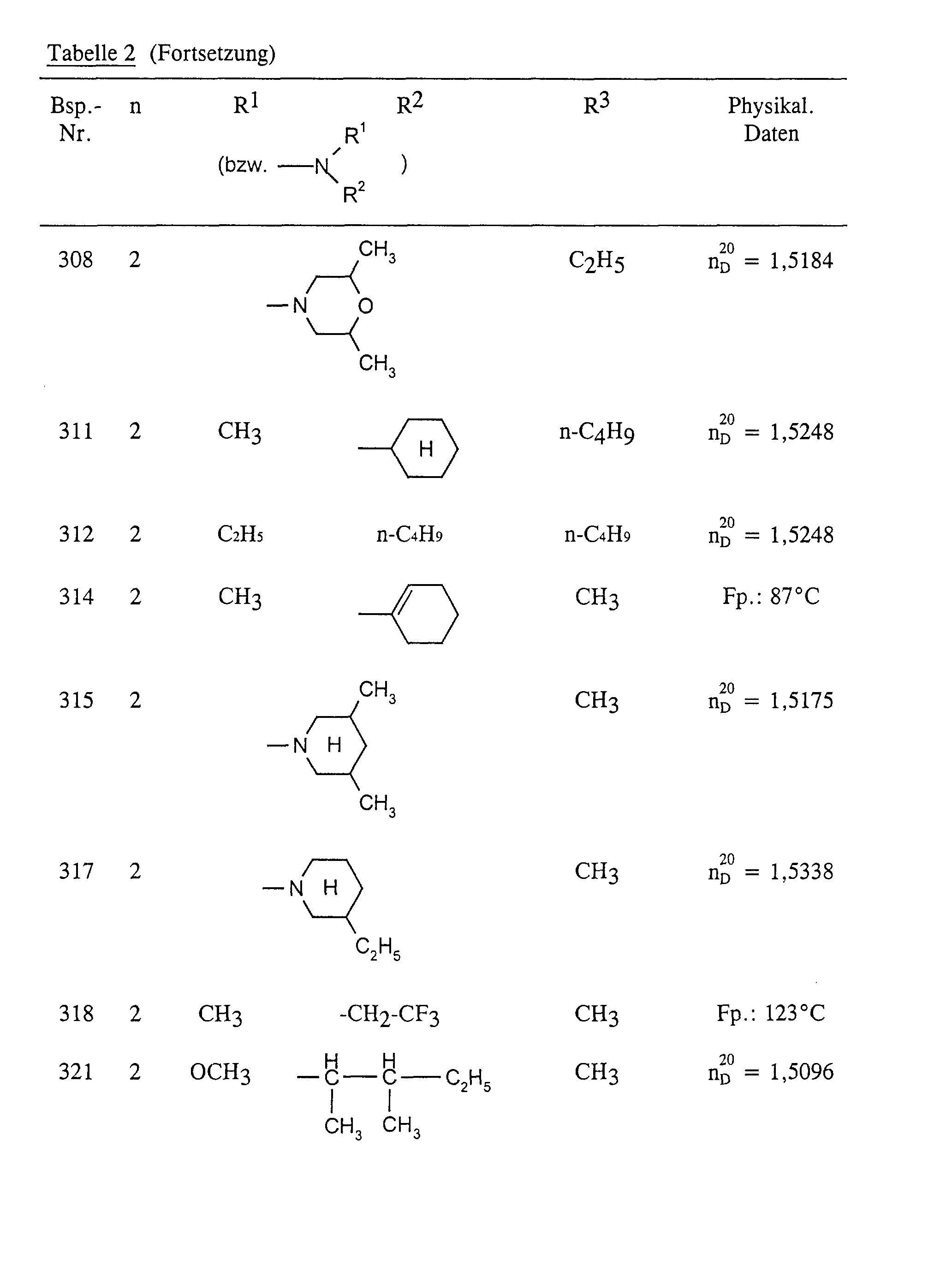

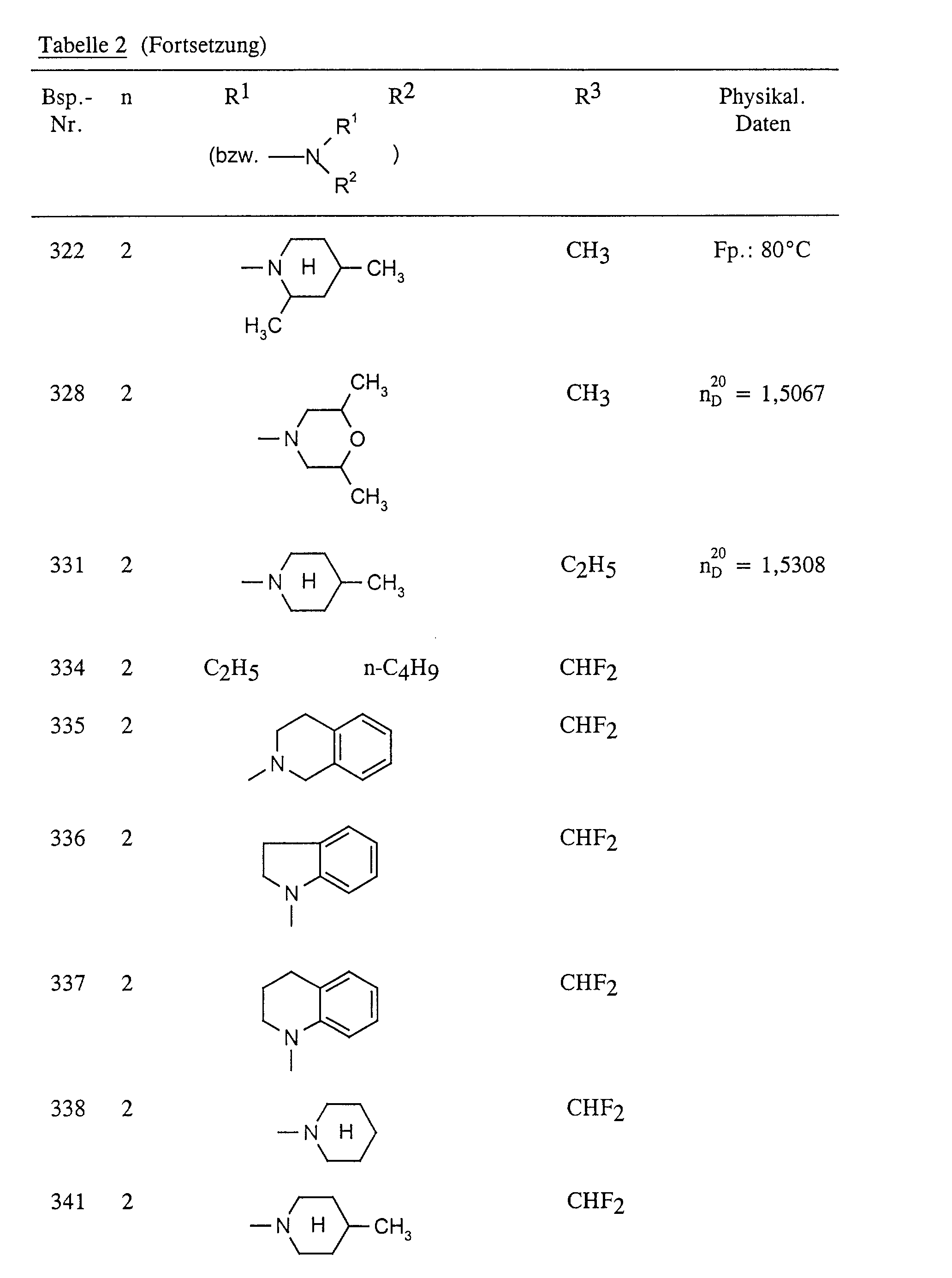

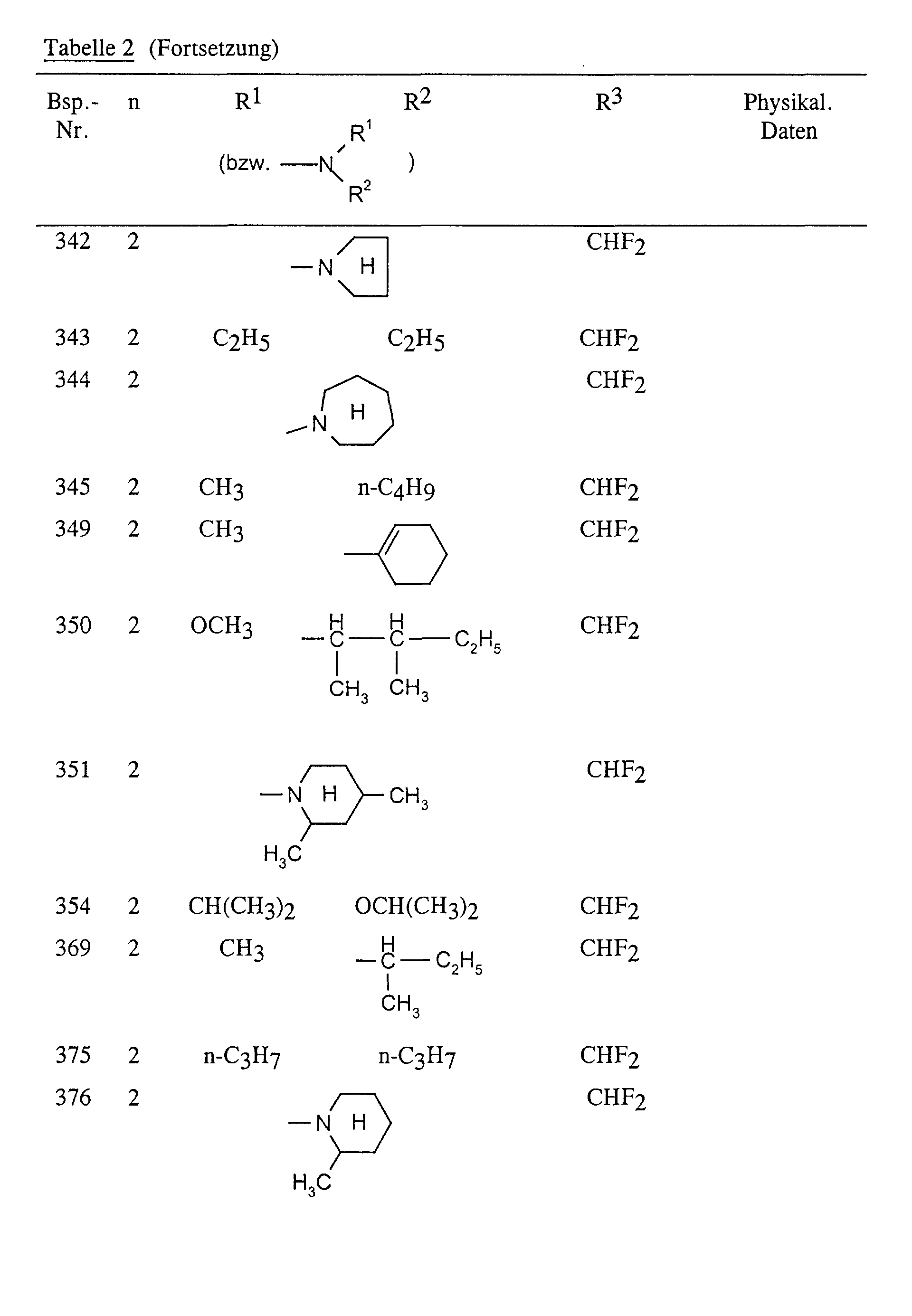

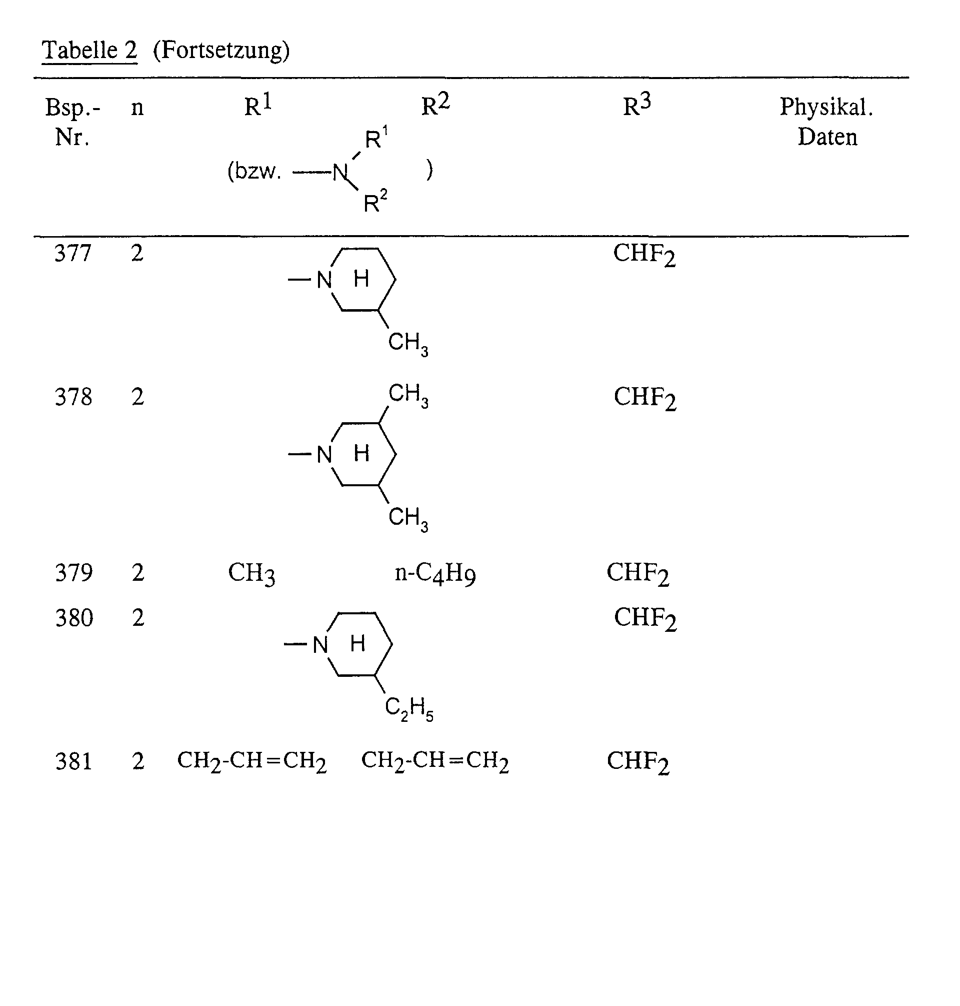

Ausgangsstoffe der Formel (II):

### Beispiel (II-1)

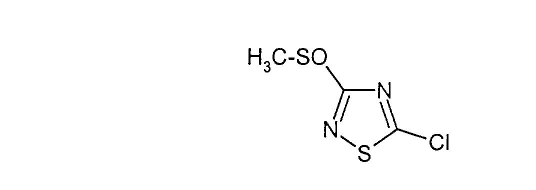

148 g (0,73 Mol) 5-Chlor-3-methylthio-1,2,4-thiadiazol werden in einer Mischung aus 500 ml Methanol und 23 g konzentrierter Schwefelsäure gelöst und zum Rückfluß erhitzt. Dann werden 90,6 g (0,8 Mol H₂O₂) einer 30%igen wäßrigen Hydrogenperoxid-Lösung zugetropft. Das Reaktionsgemisch wird 3 Stunden unter Rückfluß erhitzt. Dann wird eingeengt, der Rückstand mit Chloroform/Wasser geschüttelt, die organische Phase mit Natriumhydrogencarbonatlösung und dann mit Wasser gewaschen, mit Natriumsulfat getrocknet und filtriert. Vom Filtrat wird das Lösungsmittel im Wasserstrahlvakuum sorgfältig abdestilliert.

Man erhält 113 g (85% der Theorie) 5-Chlor-3-methylsulfinyl-1,2,4-thiadiazol als kristallines Produkt vom Schmelzpunkt 72°C.

Analog Beispiel (II-1) können beispielsweise die folgenden Verbindungen hergestellt werden:

3-Ethylsulfinyl-, 3-n-Propyl-sulfinyl-, 3-i-Propyl-sulfinyl, 3-n-Butyl-sulfinyl-, 3-i-Butyl-sulfinyl-, 3-s-Butyl-sulfinyl-, 3-Benzyl-sulfinyl, 3-(2-Chlor-benzyl)-sulfinyl-, 3-(3-Chlor-benzyl)-sulfinyl-, 3-(4-Chlor-benzyl)-sulfinyl-, 3-(3-Trifluormethylbenzyl)-sulfinyl und 3-(4-Trifluormethyl-benzyl)-sulfinyl-5-chlor-1,2,4-thiadiazol.

### Beispiel (II-2)

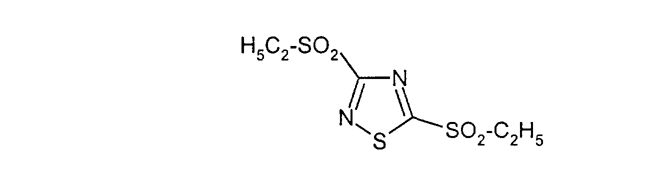

105 g (0,5 Mol) 2,5-Bis-ethylthio-1,2,4-thiadiazol und 3,5 g Natriumwolframat werden in 260 ml Essigsäure vorgelegt und bei 20°C bis 30°C innerhalb von ca. 90 Minuten mit 475 ml (5,5 Mol H₂O₂) einer 30%igen wäßrigen Hydrogenperoxid-Lösung tropfenweise versetzt. Die Reaktionsmischung wird 5 Stunden bei 20°C gerührt und dann langsam mit 2,5 Liter Wasser verdünnt. Das hierbei kristallin angefallene Produkt wird durch Absaugen isoliert.

Man erhält 100 g (74% der Theorie) 2,5-Bis-ethylsulfonyl-1,2,4-thiadiazol vom Schmelzpunkt 42°C.

Analog Beispiel (II-2) können beispielsweise auch die in der nachstehenden Tabelle 3 aufgeführten Verbindungen der Formel (II) hergestellt werden.

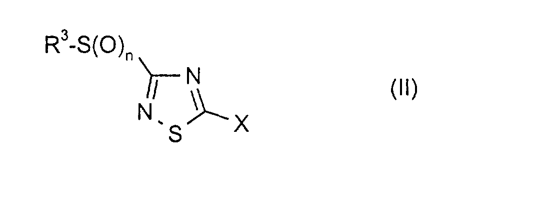

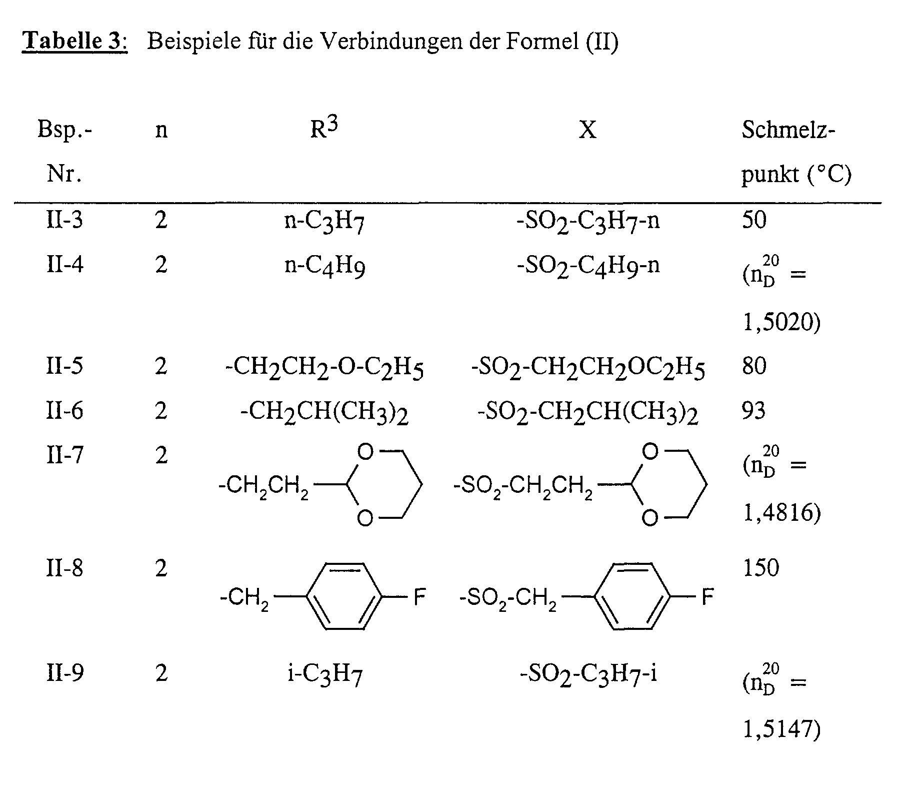

### Anwendungsbeispiel:

Im nachfolgenden Anwendungsbeispiel wird folgende Verbindung zum Vergleich herangezogen:

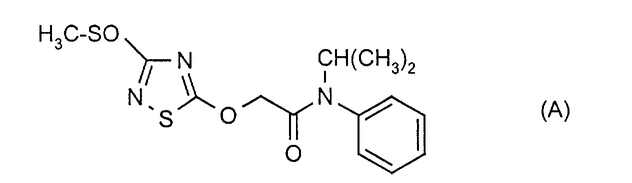

N-Isopropyl-N-phenyl-α-(3-methylsulfinyl-1,2,4-thiadiazol-5-yl-oxy)-acetamid (bekannt aus EP-A 348737/LeA 26031, vgl. Beispiel 28).

### Beispiel A

### Pre-emergence-Test

| | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach 24 Stunden wird der Boden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung

In diesem Test zeigen beispielsweise die Verbindungen gemäß den Herstellungsbeispielen 1, 5, 7, 8, 9, 11, 12, 14, 20, 21, 28, 29, 30, 31, 32, 33, 34, 35, 36, 38 und 39 bei sehr guter Verträglichkeit gegenüber Kulturpflanzen, wie z.B. Soja eine wesentlich stärkere Wirkung gegen Unkräuter als die bekannte Verbindung (A) (vgl. Tabelle A).

Weitere erfindungsgemäße Wirkstoffe und ihre Aufwandmengen, die verwendeten Testpflanzen und die Testergebnisse zeigt die nachfolgende Tabelle B; auch hierin beziehen sich die Beispielnummern auf die obigen Herstellungsbeispiele gemäß Tabelle 2.

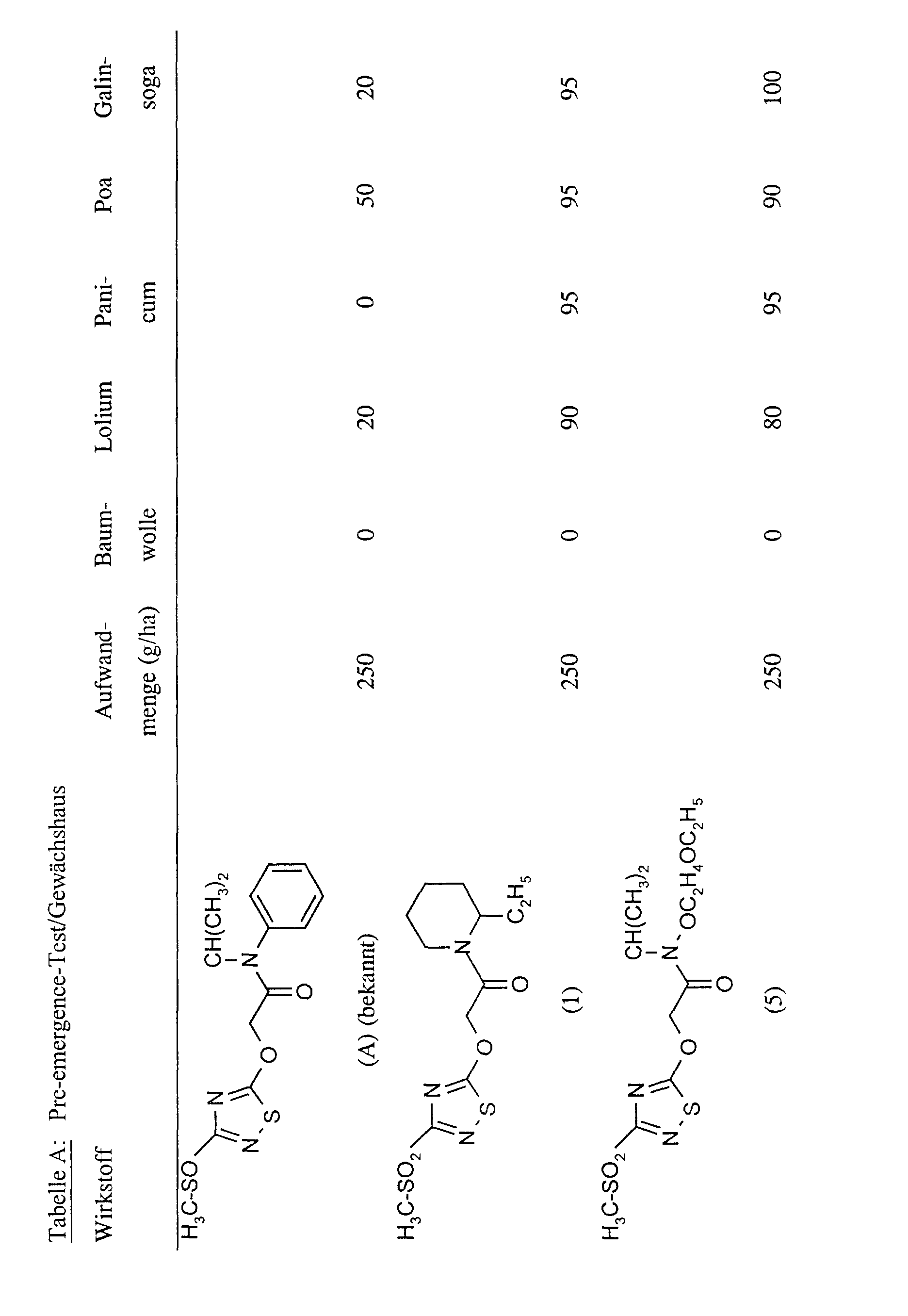

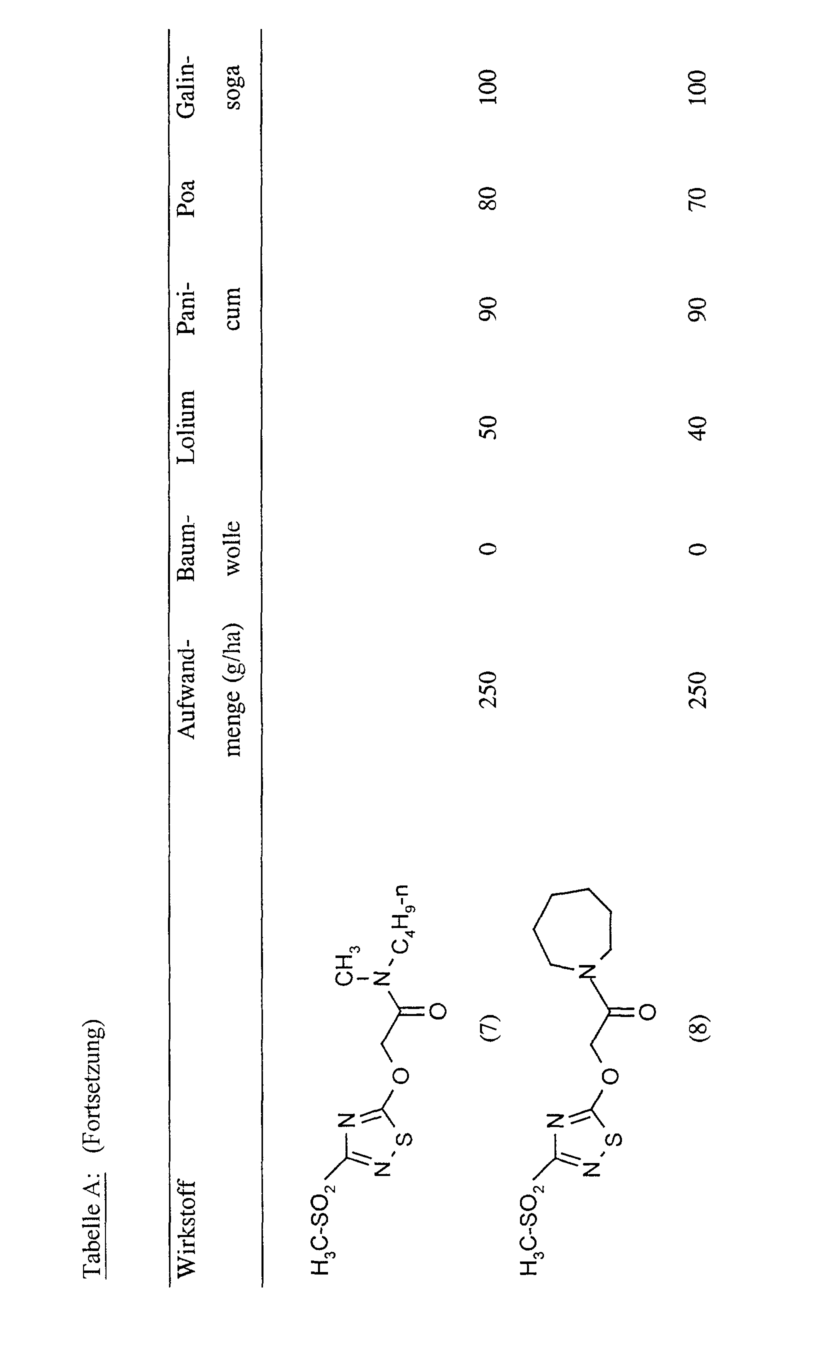

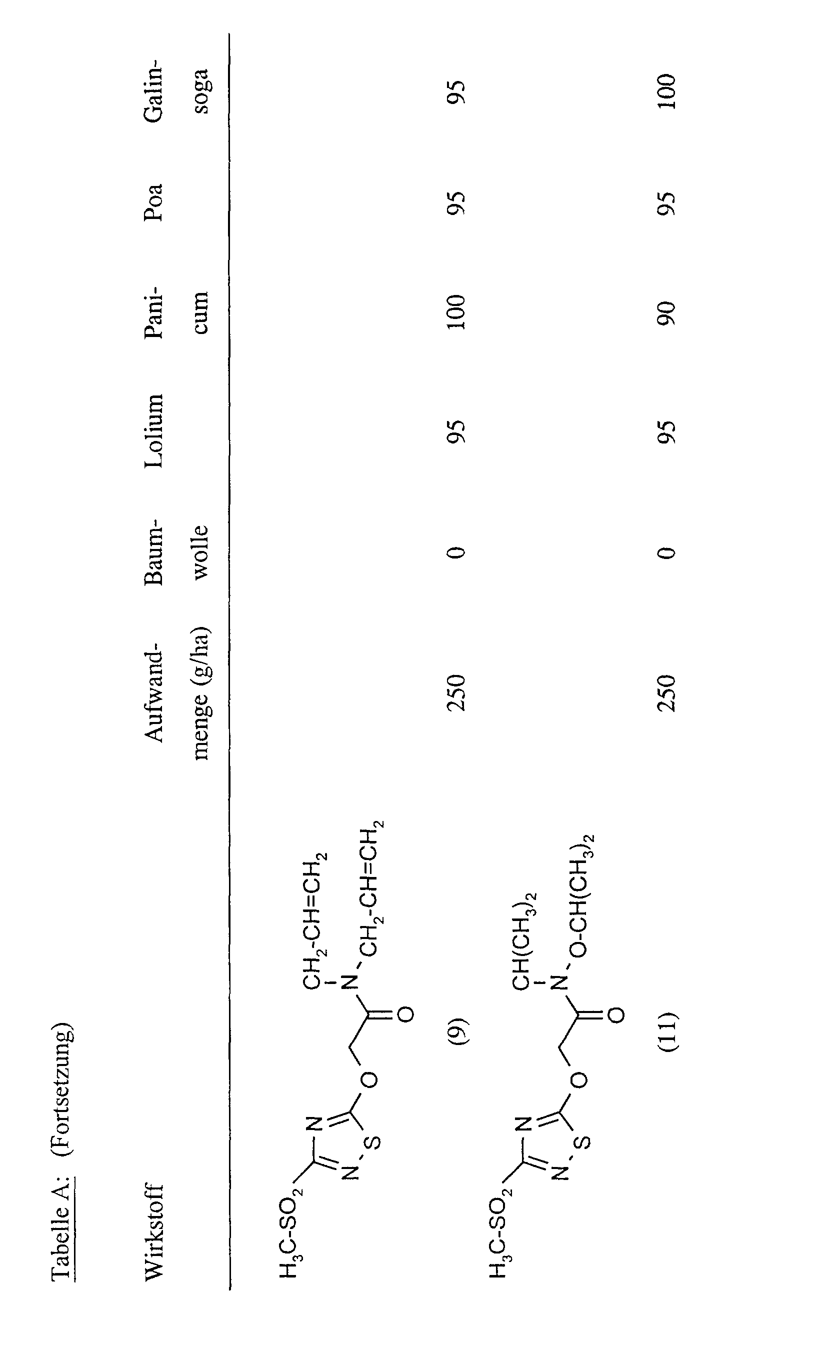

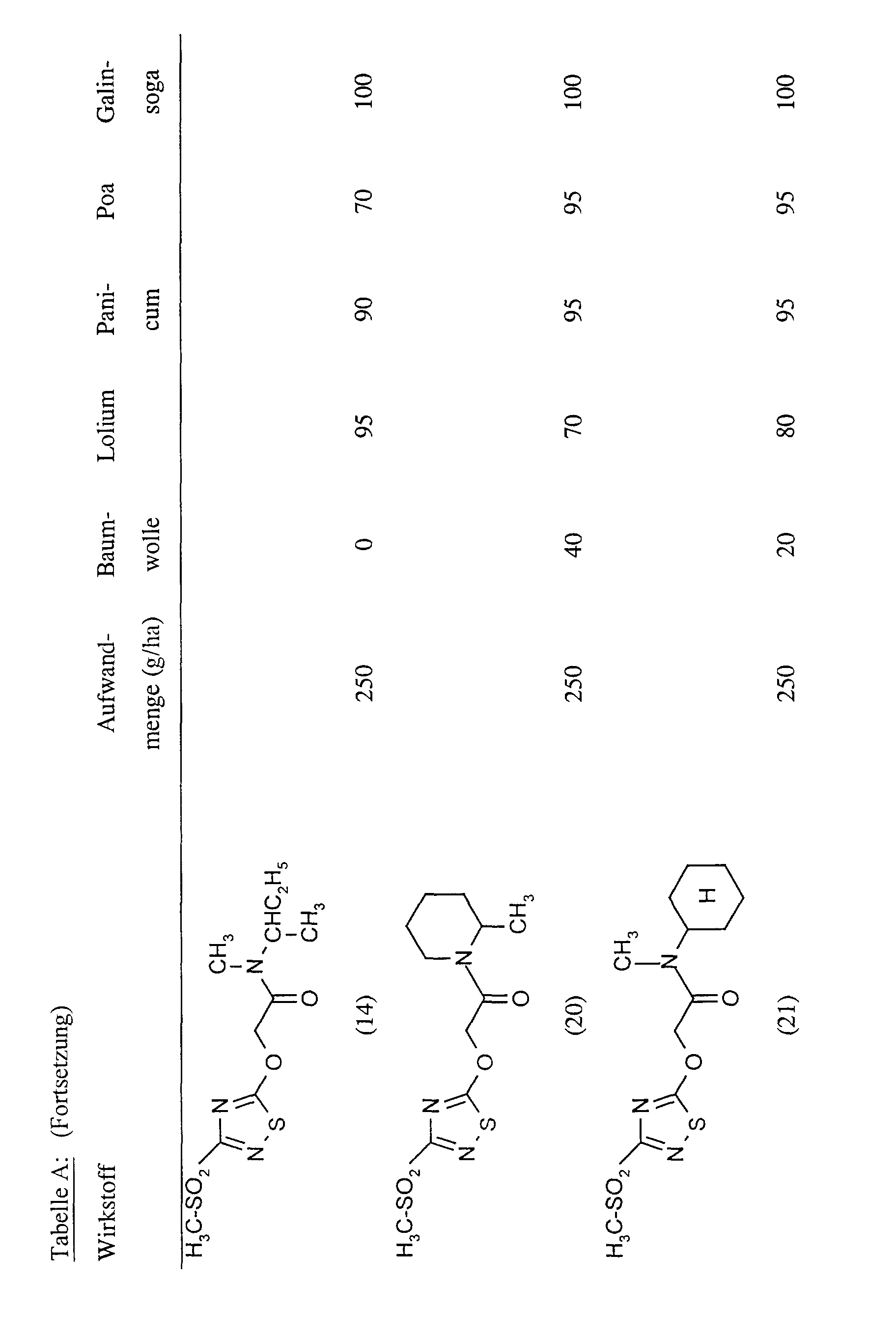

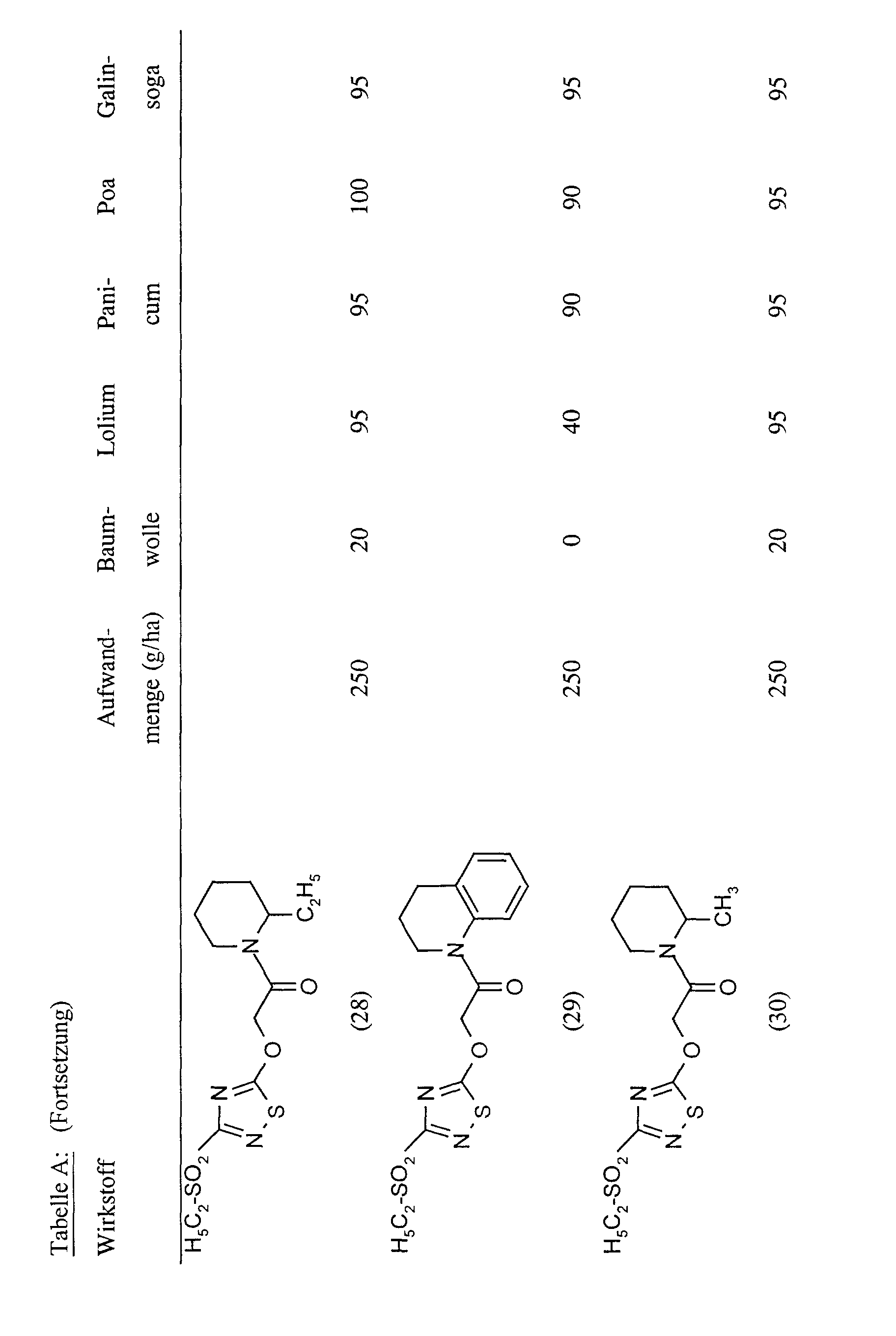

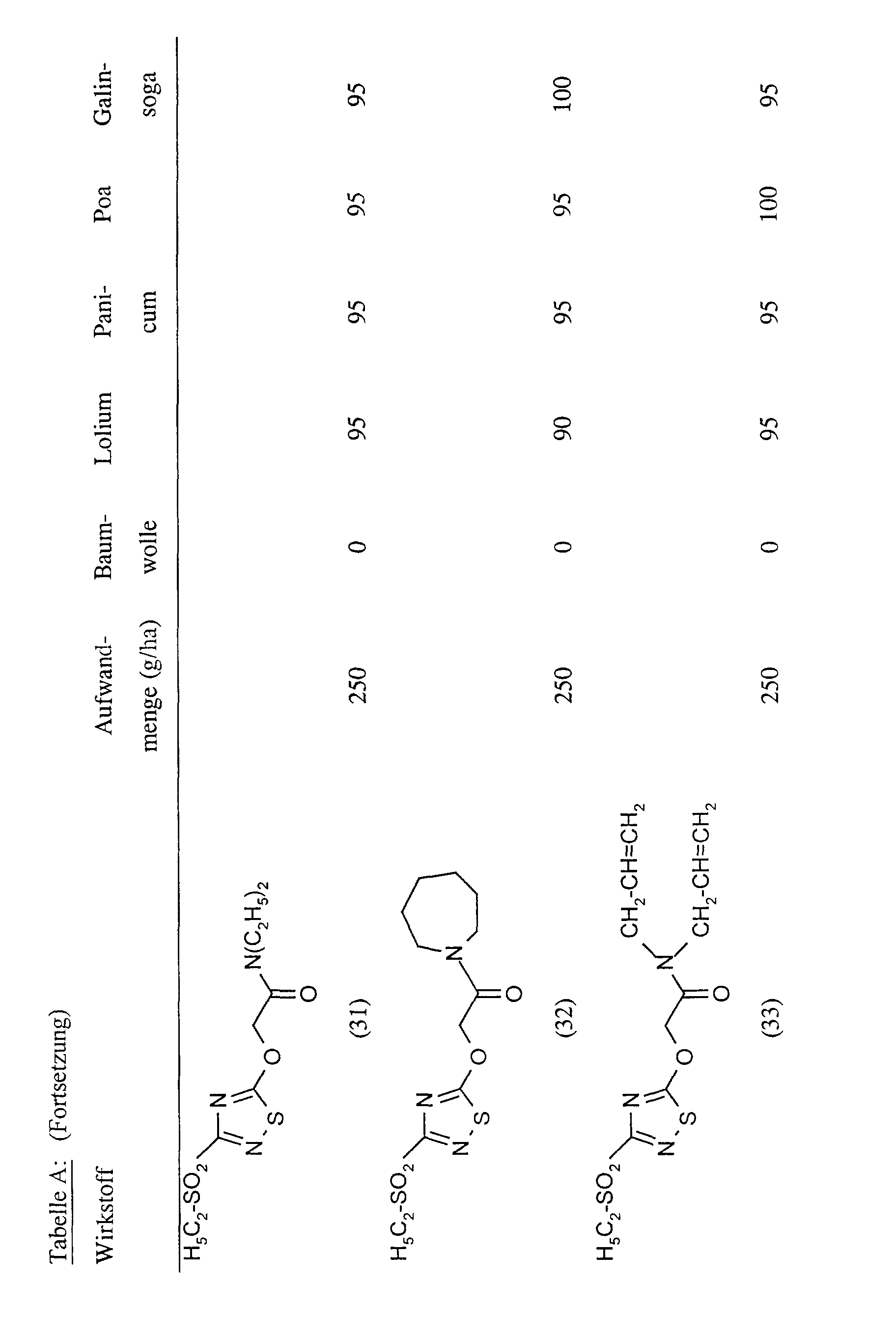

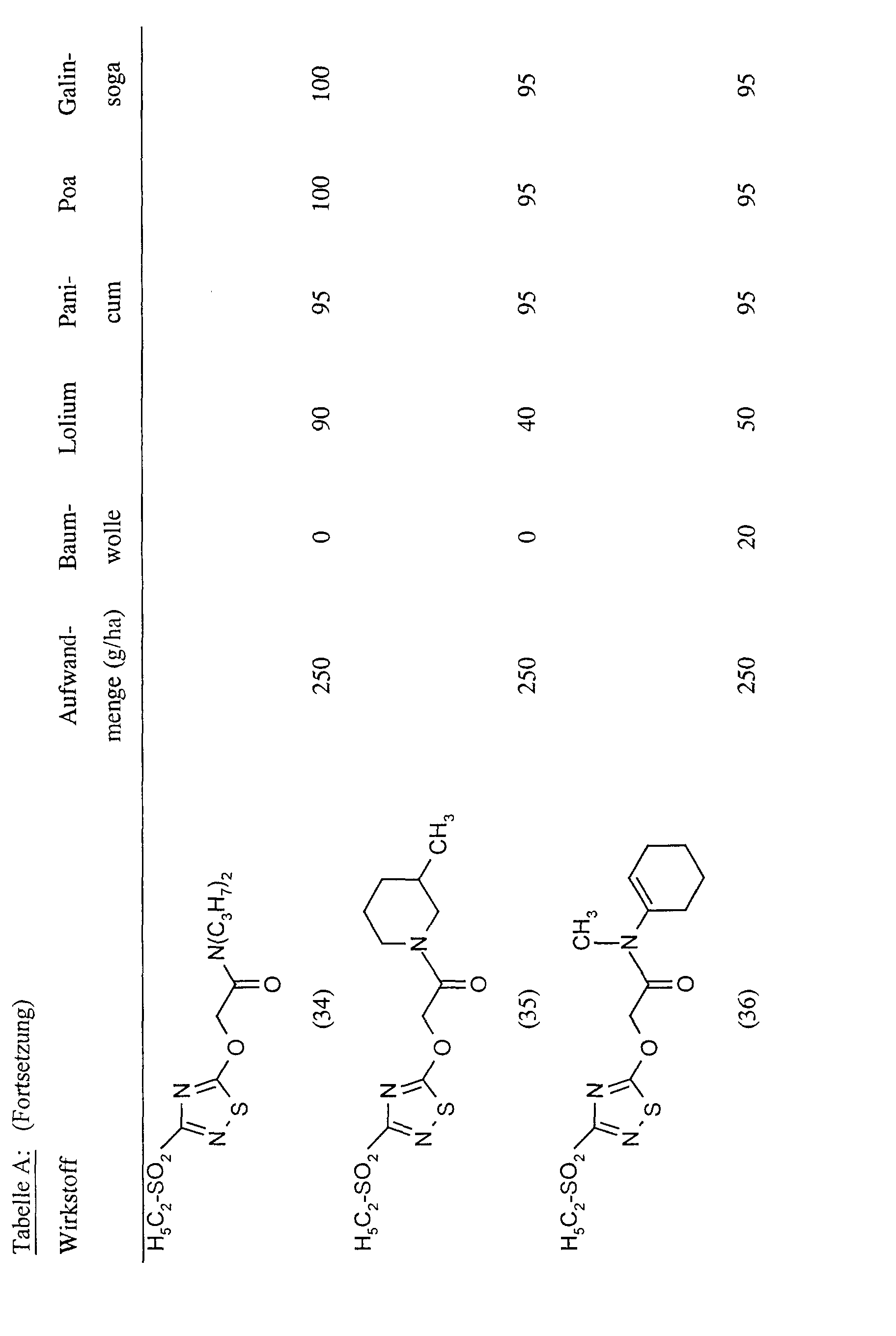

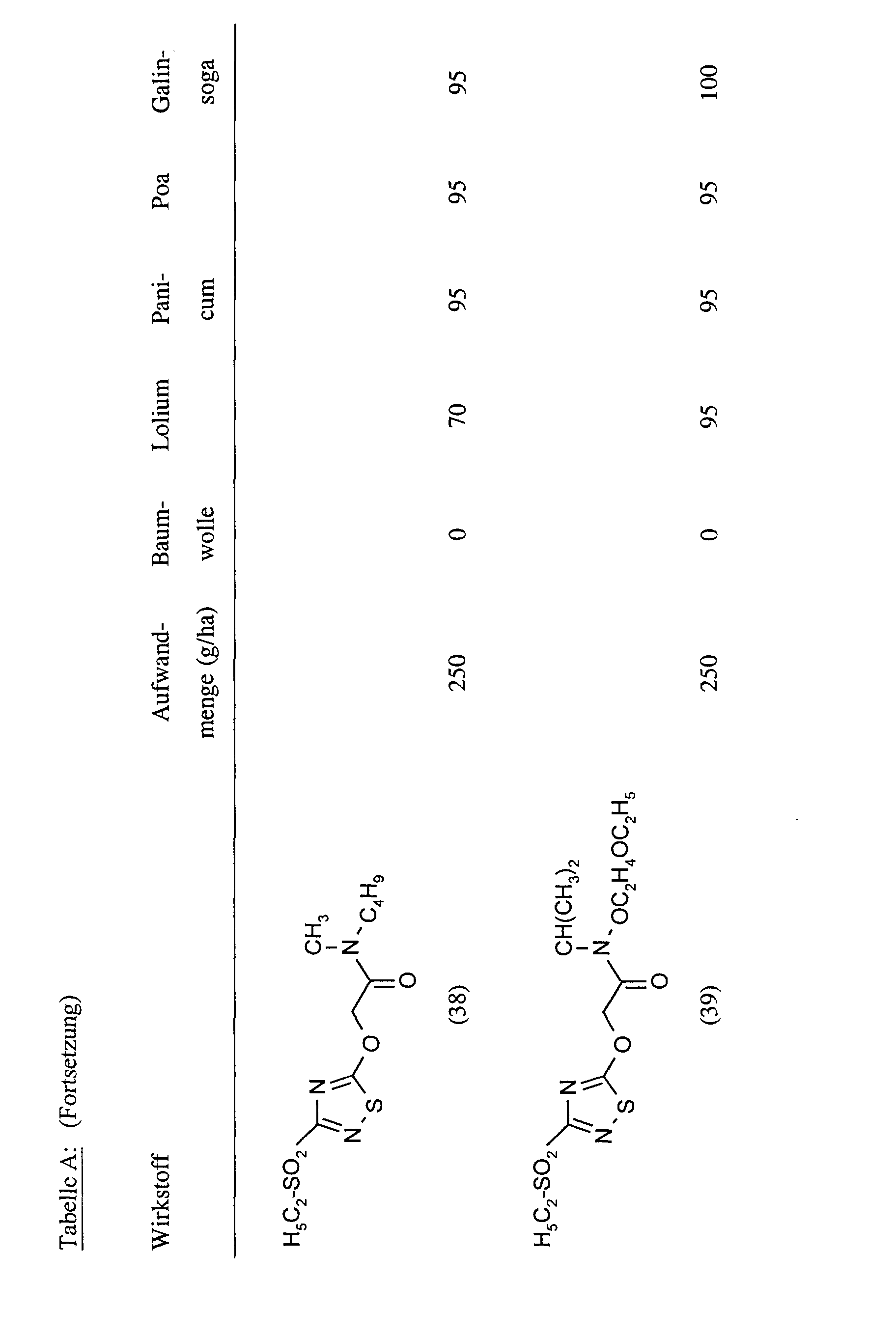

**Tabelle B:**

| Pre-emergence-Test / Gewächshaus | | | | | | | | |
|---|---|---|---|---|---|---|---|---|
| Wirkstoff Nr. | Aufwandmenge (kg/ha) | Digitaria | Echinochloa | Poa | Setaria | Amaranthus | Galinsoga | Portulaca |
| 1 | 500 | 100 | 100 | 95 | 100 | 100 | 100 | 100 |
| 5 | 500 | 95 | 100 | 100 | 100 | 100 | 100 | 100 |
| 7 | 500 | 95 | 100 | 95 | 95 | 80 | 100 | 95 |
| 8 | 500 | 100 | 100 | 95 | 90 | 90 | 100 | 100 |
| 9 | 500 | 95 | 100 | 80 | 100 | 50 | 100 | 100 |
| 11 | 500 | 95 | 100 | 95 | 100 | 95 | 100 | 95 |
| 14 | 500 | 95 | 100 | 90 | 100 | 100 | 100 | 100 |
| 16 | 500 | 95 | 95 | 80 | 70 | 80 | 100 | 100 |
| 17 | 500 | 100 | 80 | 95 | 95 | 95 | 100 | 95 |
| 20 | 250 | 95 | 95 | 95 | 95 | 95 | 100 | 80 |
| 21 | 250 | 95 | 95 | 80 | 95 | 80 | 100 | - |
| 28 | 500 | 100 | 100 | 100 | 100 | 70 | 95 | 100 |
| 29 | 500 | 95 | 95 | 90 | 90 | 70 | 95 | 95 |
| 30 | 500 | 100 | 95 | 100 | 100 | 80 | 95 | 100 |
| 31 | 500 | 100 | 100 | 100 | 100 | 70 | 95 | 100 |
| 32 | 500 | 100 | 95 | 95 | 100 | 70 | 100 | 100 |
| 33 | 500 | 100 | 100 | 100 | 100 | 100 | 95 | 100 |
| 34 | 500 | 100 | 100 | 100 | 95 | 95 | 100 | 100 |
| 35 | 500 | 100 | 100 | 100 | 100 | 95 | 100 | 90 |
| 36 | 500 | 100 | 100 | 95 | 100 | 95 | 95 | 100 |

**Tabelle B:**

| (Fortsetzung) | | | | | | | | |
|---|---|---|---|---|---|---|---|---|
| Wirkstoff Nr. | Aufwandmenge (kg/ha) | Digitaria | Echinochloa | Poa | Setaria | Amaranthus | Galinsoga | Portulaca |
| 38 | 500 | 100 | 100 | 100 | 100 | 95 | 95 | 100 |
| 39 | 500 | 100 | 100 | 100 | 100 | 95 | 100 | 100 |
| 190 | 250 | 100 | 100 | 100 | 95 | 95 | 95 | 95 |
| 191 | 250 | 95 | 100 | 95 | 95 | 80 | 80 | 60 |
| 198 | 250 | 100 | 100 | 100 | 95 | 90 | 100 | 95 |
| 199 | 250 | 95 | 100 | 100 | 100 | 60 | 95 | 95 |
| 55 | 250 | 95 | 100 | 100 | 100 | 100 | 90 | 95 |
| 56 | 250 | 100 | 100 | 100 | 100 | 100 | 95 | 95 |
| 57 | 250 | 100 | 100 | 100 | 100 | 100 | 100 | 100 |
| 58 | 250 | 100 | 100 | 100 | 100 | 100 | 95 | 90 |
| 201 | 250 | 100 | 100 | 100 | 100 | 95 | 95 | 60 |
| 203 | 250 | 100 | 100 | 100 | 95 | 100 | 95 | 90 |
| 208 | 500 | 95 | 100 | 100 | 100 | 100 | 95 | 100 |
| 78 | 500 | 100 | 100 | 100 | 100 | 100 | 100 | 100 |
| 79 | 500 | 100 | 100 | 100 | 100 | 100 | 100 | 100 |
| 80 | 500 | 100 | 100 | 100 | 100 | 100 | 100 | 100 |
| 210 | 500 | 95 | 100 | 95 | 100 | 100 | 100 | 95 |
| 216 | 250 | 95 | 95 | 100 | 100 | 95 | 100 | 100 |
| 219 | 250 | 100 | 100 | 100 | 95 | - | 100 | 100 |
| 221 | 250 | 95 | 95 | 100 | 100 | 60 | 100 | 95 |
| 103 | 250 | 100 | 100 | 100 | 100 | 50 | 100 | 100 |
| 226 | 250 | 95 | 80 | 100 | 100 | 100 | 100 | 100 |
| 227 | 250 | 100 | 70 | 100 | 95 | 100 | 100 | 100 |
| 228 | 250 | 100 | 100 | 100 | 100 | 100 | 95 | 100 |

**Tabelle B:**

| (Fortsetzung) | | | | | | | | |
|---|---|---|---|---|---|---|---|---|
| Wirkstoff Nr. | Aufwandmenge (kg/ha) | Digitaria | Echinochloa | Poa | Setaria | Amaranthus | Galinsoga | Portulaca |
| 235 | 250 | 100 | 95 | 100 | 95 | 95 | 95 | 95 |
| 237 | 250 | 95 | 95 | 100 | 95 | 95 | 95 | 60 |
| 247 | 250 | 100 | 100 | 100 | 100 | 100 | 95 | 100 |
| 251 | 250 | 100 | 95 | 100 | 90 | 100 | 100 | 95 |

## Patentansprüche

1. Alkylsulfinyl- und Alkylsulfonyl-1,2,4-thiadiazolyloxyacetamide der allgemeinen Formel (I) 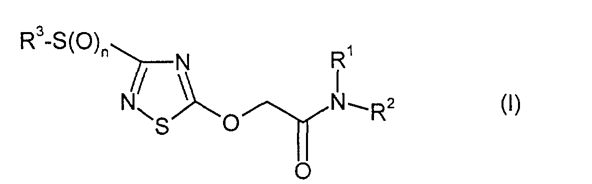 in welcher
n für die Zahlen 1 oder 2 steht,
R¹ für Wasserstoff, C₁-C₈-Alkyl (welches gegebenenfalls durch Fluor, Chlor, Cyano oder C₁-C₄-Alkoxy substituiert ist), für C₂-C₈-Alkenyl (welches gegebenenfalls durch Fluor und/oder Chlor substituiert ist), für C₂-C₈-Alkinyl oder für Benzyl steht,
R² für C₁-C₈-Alkyl (welches gegebenenfalls durch Fluor, Chlor, Cyano oder C₁-C₄-Alkoxy substituiert ist), C₂-C₈-Alkenyl (welches gegebenenfalls durch Fluor und/oder Chlor substituiert ist), für C₂-C₈-Alkinyl, für C₃-C₆-Cycloalkyl (welches gegebenenfalls durch Chlor und/oder C₁-C₃-Alkyl substituiert ist), für C₅- oder C₆-Cycloalkenyl, für C₁-C₈-Alkoxy (welches gegebenenfalls durch C₁-C₄-Alkoxy substituiert ist), oder für C₃-C₄-Alkenyloxy steht, oder
R¹ und R² zusammen mit dem Stickstoffatom, an das sie gebunden sind, einen gegebenenfalls ein- bis dreifach durch C₁-C₃-Alkyl substituierten, gesättigten oder ungesättigten fünf- bis siebengliedrigen Stickstoffheterocyclus bilden, welcher gegebenenfalls benzannelliert ist, und
R³ für C₁-C₈-Alkyl (welches gegebenenfalls durch Fluor, Chlor, Brom, Cyano, C₁-C₄-Alkoxy, Dioxolanyl oder Dioxanyl substituiert ist) oder für Phenyl oder Phenyl-C₁-C₂-alkyl (welche jeweils gegebenenfalls durch Fluor, Chlor, Brom, Cyano, Nitro, C₁-C₄-Alkyl oder C₁-C₄-Alkoxy substituiert sind) steht.

2. Verbindungen der Formel (I) gemäß Anspruch 1, **dadurch gekennzeichnet, daß** darin
n für die Zahlen 1 oder 2 steht,
R¹ für Methyl, Ethyl, n- oder i-Propyl, n-, i- oder s-Butyl, n-, i- oder s-Pentyl (welche jeweils gegebenenfalls durch Fluor, Chlor, Cyano, Methoxy oder Ethoxy substituiert sind), für Propenyl, Butenyl, Propinyl oder Butinyl steht,
R² für Methyl, Ethyl, n- oder i- Propyl, n-, i- oder s-Butyl, n-, i- oder s-Pentyl, n-, i- oder s-Hexyl (welche jeweils gegebenenfalls durch Fluor, Chlor, Cyano, Methoxy oder Ethoxy substituiert sind), für Propenyl, Butenyl, Pentenyl, Propinyl, Butinyl oder Pentinyl, für Cyclopentyl oder Cyclohexyl (welche jeweils gegebenenfalls durch Methyl und/oder Ethyl substituiert sind), für Cyclohexenyl, für Methoxy, Ethoxy, n- oder i-Propoxy, n-, i- oder s-Butoxy, n-, i-oder s-Pentyloxy (welche jeweils gegebenenfalls durch Methoxy oder Ethoxy subtituiert sind) steht, oder
R¹ und R² zusammen mit dem Stickstoffatom, an das sie gebunden sind, für gegebenenfalls ein- bis dreifach durch Methyl und/oder Ethyl substituiertes Piperidinyl, für gegebenenfalls ein- oder zweifach durch Methyl und/oder Ethyl substituiertes Pyrrolidinyl, für Perhydroazepinyl oder für 1,2,3,4-Tetrahydro(iso)-chinolinyl stehen, und
R³ für Methyl, Ethyl, n- oder i-Propyl, n-, i-, s- oder t-Butyl, n-, i-, s- oder t-Pentyl (welche jeweils gegebenenfalls durch Fluor, Chlor, Cyano, Methoxy oder Ethoxy substituiert sind) oder für Phenyl oder Benzyl (welche jeweils gegebenenfalls durch Fluor, Chlor, Cyano, Methyl oder Methoxy substituiert sind) steht.

3. Verfahren zur Herstellung von Alkylsulfinyl- und Alkylsulfonyl-1,2,4-thiadiazolyloxyacetamiden der Formel (I) gemäß Anspruch 1, **dadurch gekennzeichnet, daß** man
(a) 1,2,4-Thiadiazol-derivate der allgemeinen Formel (II) 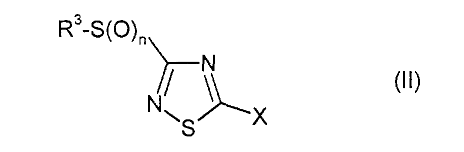 in welcher
X für Halogen oder die Gruppierung -S(O)ₙ-R³ steht und
n und R³ die in Anspruch 1 angegebene Bedeutung haben,
mit Hydroxyacetamiden der allgemeinen Formel (III) 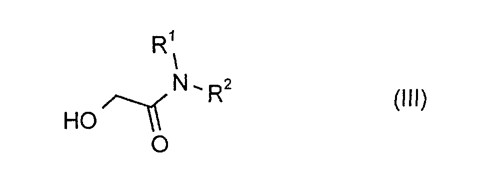 in welcher
R¹ und R² die in Anspruch 1 angegebene Bedeutung haben,
gegebenenfalls in Gegenwart eines Verdünnungsmittels, gegebenenfalls in Gegenwart eines Säurebindemittels und gegebenenfalls in Gegenwart eines Katalysators umsetzt, oder daß man
(b) Alkyl(Aryl-, Aralkyl-)thio-1,2,4-thiadiazolyloxyacetamide der allgemeinen Formel (IV) 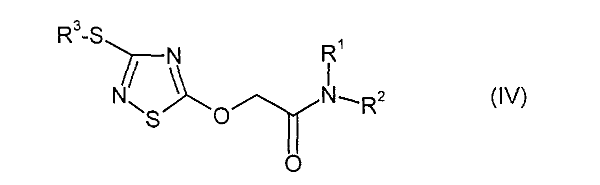 in welcher
R¹, R² und R³ die in Anspruch 1 angegebene Bedeutung haben,
mit einem Oxidationsmittel gegebenenfalls in Gegenwart eines Katalysators und gegebenenfalls in Gegenwart eines Verdünnungsmittels umsetzt.

4. Herbizide Mittel, **gekennzeichnet durch** einen Gehalt an mindestens einer Verbindung der Formel (I) gemäß Anspruch 1.

5. Verwendung von Verbindungen der allgemeinen Formel (I) gemäß Anspruch 1 zur Bekämpfung von unerwünschtem Pflanzenwachstum.

6. Verfahren zur Bekämpfung von Unkräutern, **dadurch gekennzeichnet, daß** man Verbindungen der allgemeinen Formel (I) gemäß Anspruch 1 auf die Unkräuter oder ihren Lebensraum einwirken läßt.

7. Verfahren zur Herstellung von herbiziden Mitteln, **dadurch gekennzeichnet, daß** man Verbindungen der allgemeinen Formel (I) gemäß Anspruch 1 mit Streckmitteln und/oder oberflächenaktiven Mitteln vermischt.

## Claims

1. Alkylsulphinyl- and alkylsulphonyl-1,2,4-thiadiazolyloxyacetamides of the general formula (I) 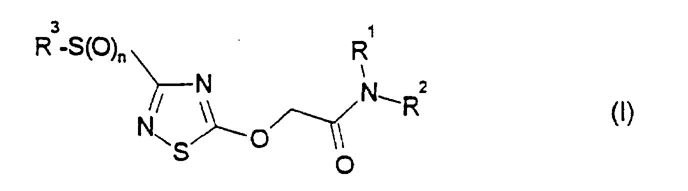 in which
n represents the numbers 1 or 2,
R¹ represents hydrogen or C₁-C₈-alkyl (which is optionally substituted by fluorine, chlorine, cyano or C₁-C₄-alkoxy), represents C₂-C₈-alkenyl (which is optionally substituted by fluorine and/or chlorine), represents C₂-C₈-alkinyl or represents benzyl,
R² represents C₁-C₈-alkyl (which is optionally substituted by fluorine, chlorine, cyano or C₁-C₄-alkoxy), or C₂-C₈-alkenyl (which is optionally substituted by fluorine and/or chlorine), represents C₂-C₈-alkinyl, represents C₃-C₆-cycloalkyl (which is optionally substituted by chlorine and/or C₁-C₃-alkyl), represents C₅- or C₆-cycloalkenyl, represents C₁-C₈-alkoxy (which is optionally substituted by C₁-C₄-alkoxy), or represents C₃-C₄-alkenyloxy, or
R¹ and R², together with the nitrogen atom to which they are bonded, form a saturated or unsaturated, five- to seven-membered nitrogen heterocycle which is optionally substituted once to three times by C₁-C₃-alkyl and which is optionally benzo fused, and
R³ represents C₁-C₈-alkyl (which is optionally substituted by fluorine, chlorine, bromine, cyano, C₁-C₄-alkoxy, dioxolanyl or dioxanyl) or represents phenyl or phenyl-C₁-C₂-alkyl (which are in each case optionally substituted by fluorine, chlorine, bromine, cyano, nitro, C₁-C₄-alkyl or C₁-C₄-alkoxy).

2. Compounds of the formula (I) according to Claim 1, **characterized in that**
n represents the numbers 1 or 2,
R¹ represents methyl, ethyl, n- or i-propyl, n-, i- or s-butyl, or n-, i- or s-pentyl (which are in each case optionally substituted by fluorine, chlorine, cyano, methoxy or ethoxy), or represents propenyl, butenyl, propinyl or butinyl,
R² represents methyl, ethyl, n- or i-propyl, n-, i or s-butyl, n-, i- or s-pentyl, or n-, i- or s-hexyl (which are in each case optionally substituted by fluorine, chlorine, cyano, methoxy or ethoxy), represents propenyl, butenyl, pentenyl, propinyl, butinyl or pentinyl, represents cyclopentyl or cyclohexyl (which are in each case optionally substituted by methyl and/or ethyl), represents cyclohexenyl, or represents methoxy, ethoxy, n- or i-propoxy, n-, i- or s-butoxy, or n-, i- or s-pentyloxy (which are in each case optionally substituted by methoxy or ethoxy), or
R¹ and R², together with the nitrogen atom to which they are bonded, represent piperidinyl which is optionally substituted one to three times by methyl and/or ethyl, represent pyrrolidinyl which is optionally substituted once or twice by methyl and/or ethyl, represent perhydroazepinyl or represent 1,2,3,4-tetrahydro(iso)-quinolinyl, and
R³ represents methyl, ethyl, n- or i-propyl, n-, i-, s- or t-butyl, n-, i-, s- or t-pentyl (which are in each case optionally substituted by fluorine, chlorine, cyano, methoxy or ethoxy) or represents phenyl or benzyl (which are in each case optionally substituted by fluorine, chlorine, cyano, methyl or methoxy).

3. Process for preparing alkylsulphinyl- and alkylsulphonyl-1,2,4-thiadiazolyloxyacetamides of the formula (I) according to Claim 1, **characterized in that**
(a)1,2,4-thiadiazole derivatives of the general formula (II) 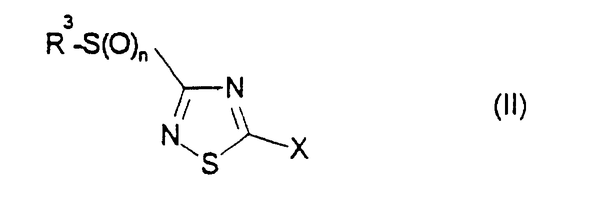 in which
X represents halogen or the grouping -S(O)ₙ-R³, and
n and R³ have the meaning given in Claim 1, are reacted with hydroxyacetamides of the general formula (III) 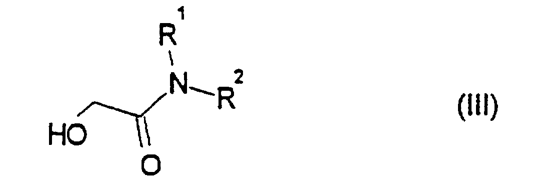 in which
R¹ and R² have the meaning given in Claim 1, where appropriate in the presence of a diluent, where appropriate in the presence of an acidbinding agent and where appropriate in the presence of a catalyst, or **in that**
(b)alkyl(aryl-, aralkyl-)thio-1,2,4-thiadiazolyloxyacetamides of the general formula (IV) 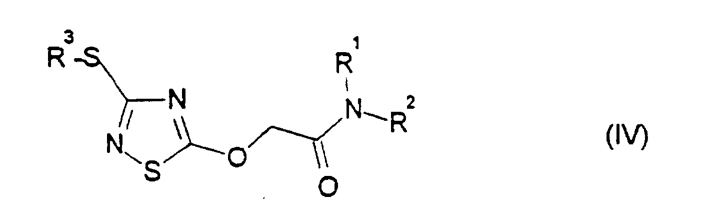 in which
R¹, R² and R³ have the meaning given in Claim 1,
are reacted with an oxidizing agent, where appropriate in the presence of a catalyst and where appropriate in the presence of a diluent.

4. Herbicidal agents, **characterized by** a content of at least one compound of the formula (I) according to Claim 1.

5. Use of compounds of the general formula (I) according to Claim 1 for controlling unwanted plant growth.

6. Process for controlling weeds, **characterized in that** compounds of the general formula (I) according to Claim 1 are allowed to act on the weeds or their habitat.

7. Process for preparing herbicidal agents, **characterized in that** compounds of the general formula (I) according to Claim 1 are mixed with extenders and/or surface-active agents.

## Revendications

1. Alkylsulfinyl- et alkylsulfonyl-1,2,4-thiadiazolyloxyacétamides de formule générale (I) 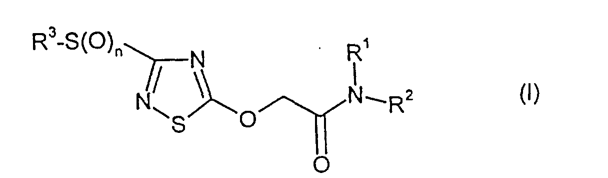 dans laquelle
n représente le nombre 1 ou 2,
R¹ est l'hydrogène, un groupe alkyle en C₁ à C₈ (qui est éventuellement substitué par du fluor, du chlore, un radical cyano ou alkoxy en C₁ à C₄), un groupe alcényle en C₂ à C₈ (qui est éventuellement substitué par du fluor et/ou du chlore), un groupe alcynyle en C₂ à C₈ ou le groupe benzyle,
R² est un groupe alkyle en C₁ à C₈ (qui est éventuellement substitué par du fluor, du chlore, un radical cyano ou alkoxy en C₁ à C₄), un groupe alcényle en C₂ à C₈ (qui est éventuellement substitué par du fluor et/ou du chlore), un groupe alcynyle en C₂ à C₈, un groupe cycloalkyle en C₃ à C₆ (qui est éventuellement substitué par du chlore et/ou un radical alkyle en C₁ à C₃), un groupe cycloalcényle en C₅ ou C₆, un groupe alkoxy en C₁ à C₈ (qui est éventuellement substitué par un radical alkoxy en C₁ à C₄) ou un groupe alcényloxy en C₃ ou C₄, ou bien
R¹ et R² forment conjointement avec l'atome d'azote auquel ils sont liés, un hétérocycle azoté pentagonal à heptagonal saturé ou non saturé qui est éventuellement substitué une à trois fois par un radical alkyle en C₁ à C₃ et éventuellement condensé au benzène, et
R³ est un groupe alkyle en C₁ à C₈ (qui est éventuellement substitué par du fluor, du chlore, du brome, un radical cyano, alkoxy en C₁ à C₄, dioxolanyle ou dioxanyle) ou un groupe phényle ou phényl-(alkyle en C₁ ou C₂) (chacun d'eux étant éventuellement substitué par du fluor, du chlore, du brome, un radical cyano, nitro, alkyle en C₁ à C₄ ou alkoxy en C₁ à C₄.

2. Composés de formule (I) suivant la revendication 1, **caractérisés en ce que** dans la formule
n représente le nombre 1 ou 2,
R¹ est un groupe méthyle, éthyle, n-propyle, isopropyle, n-butyle, isobutyle, sec.-butyle, n-pentyle, isopentyle ou sec.-pentyle (chacun d'eux étant éventuellement substitué par du fluor, du chlore, un radical cyano, méthoxy ou éthoxy), un groupe propényle, butényle, propynyle ou butynyle,
R² est un groupe méthyle, éthyle, n-propyle, isopropyle, n-butyle, isobutyle, sec.-butyle, n-pentyle, isopentyle, sec.-pentyle, n-hexyle, isohexyle, sec.-hexyle (chacun d'eux étant éventuellement substitué par du fluor, du chlore, un radical cyano, méthoxy ou éthoxy), un groupe propényle, butényle, pentényle, propynyle, butynyle ou pentynyle, un groupe cyclopentyle ou cyclohexyle (chacun d'eux étant éventuellement substitué par un radical méthyle et/ou éthyle), un groupe cyclohexényle, un groupe méthoxy, éthoxy, n-propoxy, isopropoxy, n-butoxy, isobutoxy, sec.-butoxy, n-pentyloxy, isopentyloxy, s-pentyloxy (chacun d'eux étant éventuellement substitué par un radical méthoxy ou éthoxy), ou bien
R¹ et R² forment conjointement avec l'atome d'azote auquel ils sont liés un groupe pipéridinyle éventuellement substitué une à trois fois par un radical méthyle et/ou éthyle, un groupe pyrrolidinyle éventuellement substitué une ou deux fois par un radical méthyle et/ou éthyle, un groupe perhydro-azépinyle ou un groupe 1,2,3,4-tétahydro(iso)-quinolinyle, et
R³ est un groupe méthyle, éthyle, n-propyle, isopropyle, n-butyle, isobutyle, sec.-butyle, tertio-butyle, n-pentyle, isopentyle, sec.-pentyle, tertio-pentyle, (chacun d'eux étant éventuellement substitué par du fluor, du chlore, un radical cyano, méthoxy ou éthoxy) ou un groupe phényle ou benzyle (chacun d'eux étant éventuellement substitué par du fluor, du chlore, un radical cyano, méthyle ou méthoxy.

3. Procédé de production d'alkylsulfinyl- et d'alkylsulfonyl-1,2,4-thiadiazolyloxyacétamides de formule (I) suivant la revendication 1, **caractérisé en ce que** :
(a) on fait réagir des dérivés de 1,2,4-thiadiazoles de formule générale (II) 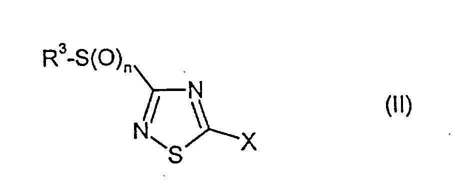 dans laquelle
x représente un halogène ou le groupement -S(O)ₙ-R³ et
n et R³ ont la définition indiquée dans la revendication 1,
avec des hydroxyacétamides de formule générale (III) 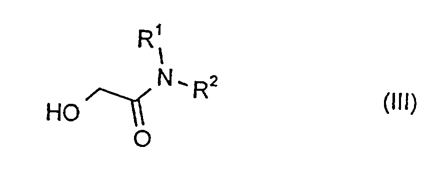 dans laquelle
R¹ et R² ont la définition indiquée dans la revendication 1, le cas échéant en présence d'un diluant, en présence éventuellement d'un accepteur d'acide et, le cas échéant, en présence d'un catalyseur, ou bien
(b) on fait réagir des alkyl(aryl-, aralkyl-)thio-1,2,4-thiadiazolyloxyacétamides de formule générale (IV) 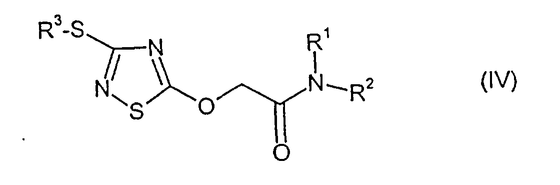 dans laquelle
R¹, R² et R³ ont la définition indiquée dans la revendication 1,
avec un agent oxydant, le cas échéant en présence d'un catalyseur et en présence éventuelle d'un diluant.

4. Compositions herbicides, **caractérisées par** une teneur en au moins un composé de formule (I) suivant la revendication 1.

5. Utilisation de composés de formule générale (I) suivant la revendication 1 pour combattre une végétation indésirable.

6. Procédé pour combattre les mauvaises herbes, **caractérisé en ce qu'**on fait agir des composés de formule générale (I) suivant la revendication 1 sur les mauvaises herbes ou sur leur milieu.

7. Procédé de préparation de compositions herbicides, **caractérisé en ce qu'**on mélange des composés de formule générale (I) suivant la revendication 1 avec des diluants et/ou des agents tensio-actifs.
